# EUROPEAN PATENT APPLICATION

(11) **EP 4 549 572 A1**
(43) Date of publication of application: **07.05.2025**
(21) Application number: 23831643.4
(22) Date of filing: 30.06.2023
(51) Int. Cl.: C12N 15/62, A61K 39/395, A61P 35/00, C07K 16/28, C07K 16/46, C12N 1/15, C12N 1/19, C12N 1/21, C12N 5/10, C12N 15/13, C12N 15/63, C12P 21/08

(54) **FUSION PROTEIN**

(30) Priority: 30.06.2022 JP 2022106054
(71) Applicant: CHIOME BIOSCIENCE INC., Shibuya-ku Tokyo 151-0071 (JP); CEINGE Biotechnologie Avanzate Franco Salvatore SCARL, 08145 Naples (IT)
(72) Inventor: NAKAMURA Koji, Tokyo 151-0071 (JP); HASHIMOTO Shuichi, Tokyo 151-0071 (JP); YOSHIOKA Asami, Tokyo 151-0071 (JP); TAKAHASHI Kota, Tokyo 151-0071 (JP); INOUE Toshikazu, Tokyo 151-0071 (JP); SANO Hitomi, Tokyo 151-0071 (JP); DE LORENZO, Claudia, 48680145 Napoli (IT); PASSARIELLO, Margherita, 48680145 Napoli (IT)
(74) Representative: J A Kemp LLP
(86) International application number: PCT/JP2023/024487
(87) International publication number: WO 2024/005204

(57) **Abstract**

Provided are a novel protein that is a trispecific tribody with an increased anti-tumor efficacy and the like. The invention relates to a fusion protein including two different first and second chains, in which three combinatorial VH/VL binding domains formed within the fusion protein have any one selected from the following binding abilities (provided that the three combinatorial VH/VL binding domains have different binding abilities from one another): (i) ability to bind to 5T4; (ii) ability to bind to a CD3 receptor complex on T cells; and (iii) ability to bind to PD-1, PD-L1, or LAG3.

## Description

### Technical Field

The present invention relates to a fusion protein and a use application or the like thereof. Specifically, the present invention relates to a fusion protein having ability to bind to (i) 5T4, (ii) a CD3 receptor complex, and (iii) PD-1, PD-L1 or LAG3, and a use application or the like thereof.

### Background Art

Cd3-based T-cell engager (TCE) is an antibody or antibody-like protein that simultaneously binds with one arm to a tumor-associated antigen (TAA) on cancer cells and with the other arm to CD3 complex on a T-cell to form a TCR-independent artificial immune synapse and circumvent HLA restriction in T cell-dependent immune response. TCE is one of the most attractive modalities to treat cancers resistant to conventional therapies. In the field of hematological malignancies, Blinatumomab is a Bi-specific T cell engager (TCE) (BiTE) that simultaneously targets CD19 antigen on the acute lymphoblastic leukemia (ALL) and CD3 on T- cells to induce efficient killing of leukemia cells (FDA approval in 2014). On the other hand, significant challenges in treating solid tumors with TCEs are represented by the immunosuppressive tumor microenvironment (TME). Solid tumors recruit immunosuppressive cells such as myeloid-derived suppressor cells (MDSCs), tumor-associated macrophages (TAMs), and regulatory T-cells (Tregs) to form TME, all of which inhibit the activity of cytotoxic T-cells.

Therefore, the most effective use of TCEs in solid tumors will likely require the use of TCEs combined with agents such as immune checkpoint inhibitors that help to overcome the immunosuppressive TME and render an immune excluded or immune desert "cold" tumor into an inflamed "hot" one (see, for example, Non Patent Literature 1). Ideally, a single molecule could be designed that is able to have both the functions of TCE and of immune check point inhibitor, so that this novel construct will allow to overcome the immunosuppressive TME in solid tumors and to lower the cost of production with respect to that of two separate drugs to be combined.

The oncofetal tumor-associated antigen 5T4, also known as TPBG or WAIF1, is a 72-kDa cell surface glycoprotein containing eight leucine-rich repeats. 5T4 is expressed in fetal trophoblasts as early as 9 weeks of gestation. The expression of 5T4 in normal adults is restricted to a few specialized epithelial cell types but not detected in the liver, lung, bronchus, heart, testis, ovary, brain or muscles.

On the other hand, it has been reported that 5T4 is expressed in a wide variety of cancer types including ovarian, colon, cervical, gastric and lung cancers (see, for example, Non Patent Literature 2 to 6). In addition, its expression has been observed also on bladder, endometrium, oesophagus, pancreas, stomach and testicular non-seminomatous germ cell tumours. Choriocarcinomas and placental site trophoblastic tumours were also positive for 5T4 (see, for example, Non Patent Literature 7). Therefore, 5T4 has been suggested as a suitable target antigen for targeted cancer therapy (see, for example, Non Patent Literature 8 and 9)

Bi-specific antibodies that engage 5T4 on tumor cells and CD3 expressed on T cells may represent an interesting strategy to redirect cytotoxic T cells against cancer cells for inducing efficient killing of cancer cells. There are some bi-specific antibodies targeting 5T4 on the tumor cells and CD3 or 4-1BB which is a co-stimulatory molecule on T cells to treat solid tumors in clinical trials. However, to date, no anti-5T4 immunotherapy has been approved for treating cancer.

As an aside, the tribody format is a multi-specific antibody including a Fab domain as a scaffold, in which Fab fragment of H-chain (V_{H}+CH) and L-chain (V_{L}+C_{L}) can naturally heterodimerize *in vivo,* and additional functions such as scFvs that can be incorporated onto the scaffold. Among the tribodies, Tb535H is a bi-specific tribody, made up of a Fab and a scFv domain both targeting 5T4 (bivalent binding) and another scFv targeting CD3 (monovalent binding). It is known that it can exert certain anti-tumor efficacy (see, for example, Patent Literature 1).

### Citation List

### Patent Literature

Patent Literature 1: WO 2016/097408 A1
Patent Literature 2: WO 2019/180201 A2
Patent Literature 2: WO 99/37791

### Non Patent Literature

Non Patent Literature 1: Hegde PS, Chen DS. Top 10 challenges in cancer immunotherapy. Immunity. (2020) 52:17-35. doi: 10.1016/j.immuni.2019.12.011
Non Patent Literature 2: Int J Gynecol Cancer. 1995;5:269-274
Non Patent Literature 3: Starzynska T, et al. Prognostic significance of 5T4 oncofetal antigen expression in colorectal carcinoma. Br J Cancer. 1994;69:899- 902
Non Patent Literature 4: Jones H, et al., Investigation of expression of 5T4 antigen in cervical cancer. Br J Cancer. 1990;6:69-100
Non Patent Literature 5: Starzynska T, et al. The expression of 5T4 antigen in colorectal and gastric carcinoma. Br J Cancer. 1992;66:867-869
Non Patent Literature 6: Forsberg G, et al. Therapy of human non-small-cell lung carcinoma using antibody targeting of modified superantigen. Br J Cancer. 2001 ;85:129-136
Non Patent Literature 7: Br J Cancer. 1990 Jan;61(1):89-95. doi: 10.1038/bjc.1990.20.
Non Patent Literature 8: Woods AM, et al. Characterization of the murine 5T4 oncofoetal antigen: a target for immunotherapy in cancer. Biochem J. 2002;366:353-365
Non Patent Literature 9: Hole N, et al. Isolation and characterization of 5T4, A tumor-associated antigen. Int J Cancer. 1990;45:179-184
Non Patent Literature 10: Sasso, et al. MABS 2018, VOL. 10, NO. 7, 1060-1072
Non Patent Literature 11: Passariello et al. IJMS 2022, 23, 3466

### Summary of Invention

### Technical Problem

Under such circumstances, it has been desired to develop a novel protein as a multispecific tribody with further enhanced anti-tumor efficacy.

### Solution to Problem

The present invention has been made in consideration of the above situation, and provides a fusion protein and an application thereof (such as a pharmaceutical composition) described below.

[1] A fusion protein comprising two different first and second chains,
   wherein the first chain comprises two antibody variable regions VH1 and VH2, one antibody variable region VL2, and one antibody constant region CH1 or CL,
   the second chain comprises two antibody variable regions VL1 and VL3, one antibody variable region VH3, and one antibody constant region CL or CH1,
   the first and second chains contain a heterodimeric interaction (preferably the first and second chains contain the heterodimeric interaction between CH1 of one chain and the CL domain of the other chain), and
   a VH1/VL1 binding domain, a VH2/VL2 binding domain, and a VH3/VL3 binding domain, which are three combinatorial VH/VL binding domains formed within the fusion protein, have any binding ability selected from the following (provided that the three combinatorial VH/VL binding domains have different binding abilities from one another):
      (i) ability to bind to 5T4;
      (ii) ability to bind to a CD3 receptor complex; and
      (iii) ability to bind to PD-1, PD-L1, or LAG3.
[2] The fusion protein according to [1], which is a heterodimeric protein comprising the first chain and the second chain.
[3] The fusion protein according to [1] or [2], wherein the two different chains comprise:
   a) a first chain:
      VH (5T4)-CH1-L1-VH (CD3)-L2-VL (CD3) or
      VH (5T4)-CH1-L1-VL (CD3)-L2-VH (CD3) combined with a second chain:
         VL (5T4)-CL-L1-VH (X)-L2-VL (X) or
         VL (5T4)-CL-L1-VL (X)-L2-VH (X);
   b) a first chain:
      VH (5T4)-CH1-L1-VH (X)-L2-VL (X) or
      VH (5T4)-CH1-L1-VL (X)-L2-VH (X) combined with a second chain:
         VL (5T4)-CL-L1-VH (CD3)-L2-VL (CD3) or
         VL (5T4)-CL-L1-VL (CD3)-L2-VH (CD3);
   c) a first chain:
      VL (5T4)-CH1-L1-VH (CD3)-L2-VL (CD3) or
      VL (5T4)-CH1-L1-VL (CD3)-L2-VH (CD3) combined with a second chain:
         VH (5T4)-CL-L1-VH (X)-L2-VL (X) or
         VH (5T4)-CL-L1-VL (X)-L2-VH (X);
   d) a first chain:
      VL (5T4)-CH1-L1-VH (X)-L2-VL (X) or
      VL (5T4)-CH1-L1-VL (X)-L2-VH (X) combined with a second chain:
         VH (5T4)-CL-L1-VH (CD3)-L2-VL (CD3) or
         VH (5T4)-CL-L1-VL (CD3)-L2-VH (CD3);
   e) a first chain:
      VH (CD3)-CH1-L1-VH (5T4)-L2-VL (5T4) or
      VH (CD3)-CH1-L1-VL (5T4)-L2-VH (5T4) combined with a second chain:
         VL (CD3)-CL-L1-VH (X)-L2-VL (X) or
         VL (CD3)-CL-L1-VL (X)-L2-VH (X);
   f) a first chain:
      VH (CD3)-CH1-L1-VH (X)-L2-VL (X) or
      VH (CD3)-CH1-L1-VL (X)-L2-VH (X) combined with a second chain:
         VL (CD3)-CL-L1-VH (5T4)-L2-VL (5T4) or
         VL (CD3)-CL-L1-VL (5T4)-L2-VH (5T4);
   g) a first chain:
      VL (CD3)-CH1-L1-VH (5T4)-L2-VL (5T4) or
      VL (CD3)-CH1-L1-VL (5T4)-L2-VH (5T4) combined with a second chain:
         VH (CD3)-CL-L1-VH (X)-L2-VL (X) or
         VH (CD3)-CL-L1-VL (X)-L2-VH (X);
   h) a first chain:
      VL (CD3)-CH1-L1-VH (X)-L2-VL (X) or
      VL (CD3)-CH1-L1-VL (X)-L2-VH (X) combined with a second chain:
         VH (CD3)-CL-L1-VH (5T4)-L2-VL (5T4) or
         VH (CD3)-CL-L1-VL (5T4)-L2-VH (5T4);
   i) a first chain:
      VH (X)-CH1-L1-VH (5T4)-L2-VL (5T4) or
      VH (X)-CH1-L1-VL (5T4)-L2-VH (5T4) combined with a second chain:
         VL (X)-CL-L1-VH (CD3)-L2-VL (CD3) or
         VL (X)-CL-L1-VL (CD3)-L2-VH (CD3);
   j) a first chain:
      VH (X)-CH1-L1-VH (CD3)-L2-VL (CD3) or
      VH (X)-CH1-L1-VL (CD3)-L2-VH (CD3) combined with a second chain:
         VL (X)-CL-L1-VH (5T4)-L2-VL (5T4) or
         VL (X)-CL-L1-VL (5T4)-L2-VH (5T4);
   k) a first chain:
      VL (X)-CH1-L1-VH (5T4)-L2-VL (5T4) or
      VL (X)-CH1-L1-VL (5T4)-L2-VH (5T4) combined with a second chain:
         VH (X)-CL-L1-VH (CD3)-L2-VL (CD3) or
         VH (X)-CL-L1-VL (CD3)-L2-VH (CD3); or
   l) a first chain:
      VL (X)-CH1-L1-VH (CD3)-L2-VL (CD3) or
      VL (X)-CH1-L1-VL (CD3)-L2-VH (CD3) combined with a second chain:
         VH (X)-CL-L1-VH (5T4)-L2-VL (5T4) or
         VH (X)-CL-L1-VL (5T4)-L2-VH (5T4)
            [where in a) to l) above,
         VH and VL are antibody variable regions,
         VH (5T4) and VL (5T4) are antibody variable regions for 5T4,
         VH (CD3) and VL (CD3) are antibody variable regions for the CD3 receptor complex,
         VH (X) and VL (X) are antibody variable regions for PD-1, PD-L1, or LAG3,
         CH1 and CL are antibody constant regions, and
         L1 and L2 are linkers].
[4] The fusion protein according to [1] or [2], wherein amino acid sequences of complementarity determining regions (CDRs) 1, 2, and 3 in VH1, VH2, and VH3 consist respectively of:
   amino acid sequences set forth in SEQ ID NOS: 3, 4, and 5;
   amino acid sequences set forth in SEQ ID NOS: 9, 10, and 11;
   amino acid sequences set forth in SEQ ID NOS: 24, 25, and 26 or 27;
   amino acid sequences set forth in SEQ ID NOS: 37, 38, and 39;
   amino acid sequences set forth in SEQ ID NOS: 47, 48 or 49, and 50; or
   amino acid sequences set forth in SEQ ID NOS: 58, 59 or 49, and 60, and
   wherein amino acid sequences of CDRs 1, 2, and 3 in VL1, VL2, and VL3 consist respectively of:
   amino acid sequences set forth in SEQ ID NOS: 19, 20, and 21;
   amino acid sequences set forth in SEQ ID NOS: 14, 15, and 16;
   amino acid sequences set forth in SEQ ID NOS: 29, 30, and 31, 32, or 33;
   amino acid sequences set forth in SEQ ID NOS: 41, 42, and 43;
   amino acid sequences set forth in SEQ ID NOS: 52, 53, and 54; or
   amino acid sequences set forth in SEQ ID NOS: 62, 63, and 64.
[5] The fusion protein according to [1] or [2], wherein the amino acid sequences of VH1, VH2, and VH3 differ from each other, and each consist of an amino acid sequence set forth in SEQ ID NO: 88, 90, 8, 92, 23, 78, 46, 81, 57, 109, 82, 83, 84, or 36, comprise the amino acid sequence, or have at least 90% identity with the amino acid sequence, and
   wherein the amino acid sequences of VL1, VL2, and VL3 differ from each other, and each consist of an amino acid sequence set forth in SEQ ID NO: 89, 91, 13, 93, 28, 79, 80, 51, 61, 85, 86, 87, or 40, comprise the amino acid sequence, or have at least 90% identity with the amino acid sequence.
[6] The fusion protein according to [3], wherein amino acid sequences of complementarity determining regions (CDRs) 1, 2, and 3 in VH (5T4) consist respectively of amino acid sequences set forth in SEQ ID NOS: 3, 4, and 5, and
   wherein amino acid sequences of CDRs 1, 2, and 3 in VL(5T4) consist respectively of:
   amino acid sequences set forth in SEQ ID NOS: 19, 20, and 21.
[7] The fusion protein according to [3], wherein amino acid sequences of complementarity determining regions (CDRs) 1, 2, and 3 in VH (CD3) consist respectively of amino acid sequences set forth in SEQ ID NOS: 9, 10, and 11, and
   wherein amino acid sequences of CDRs 1, 2, and 3 in VL(CD3) consist respectively of: amino acid sequences set forth in SEQ ID NOS: 14, 15, and 16.
[8] The fusion protein according to [3], wherein amino acid sequences of complementarity determining regions (CDRs) 1, 2, and 3 in VH (X) (where X is PD-1) consist respectively of amino acid sequences set forth in SEQ ID NOS: 24, 25, and 26 or 27, and
   wherein amino acid sequences of CDRs 1, 2, and 3 in VL (X) (where X is PD-1) consist respectively of amino acid sequences set forth in SEQ ID NOS: 29, 30, and 31, 32, or 33.
[9] The fusion protein according to [3], wherein amino acid sequences of complementarity determining regions (CDRs) 1, 2, and 3 in VH (X) (where X is PD-L1) consist respectively of amino acid sequences set forth in SEQ ID NOS: 47, 48 or 49, and 50, and
   wherein amino acid sequences of CDRs 1, 2, and 3 in VL (X) (where X is PD-L1) consist respectively of amino acid sequences set forth in SEQ ID NOS: 52, 53, and 54.
[10] The fusion protein according to [3], wherein amino acid sequences of complementarity determining regions (CDRs) 1, 2, and 3 in VH (X) (where X is PD-L1) consist respectively of amino acid sequences set forth in SEQ ID NOS: 58, 59 or 49, and 60, and
   wherein amino acid sequences of CDRs 1, 2, and 3 in VL (X) (where X is PD-L1) consist respectively of amino acid sequences set forth in SEQ ID NOS: 62, 63, and 64.
[11] The fusion protein according to [3], wherein amino acid sequences of complementarity determining regions (CDRs) 1, 2, and 3 in VH (X) (where X is LAG3) consist respectively of amino acid sequences set forth in SEQ ID NOS: 37, 38, and 39, and
   wherein amino acid sequences of CDRs 1, 2, and 3 in VL (X) (where X is LAG3) consist respectively of amino acid sequences set forth in SEQ ID NOS: 41, 42, and 43.
[12] The fusion protein according to [3], wherein the amino acid sequence of VH (5T4) consists of an amino acid sequence set forth in SEQ ID NO: 88 or 90, comprises the amino acid sequence, or has at least 90% identity with the amino acid sequence, and
   wherein the amino acid sequence of VL (5T4) consists of an amino acid sequence set forth in SEQ ID NO: 89 or 91, comprises the amino acid sequence, or has at least 90% identity with the amino acid sequence.
[13] The fusion protein according to [3], wherein the amino acid sequence of VH (CD3) consists of an amino acid sequence set forth in SEQ ID NO: 8 or 92, comprises the amino acid sequence, or has at least 90% identity with the amino acid sequence, and
   wherein the amino acid sequence of VL (CD3) consists of an amino acid sequence set forth in SEQ ID NO: 13 or 93, comprises the amino acid sequence, or has at least 90% identity with the amino acid sequence.
[14] The fusion protein according to [3], wherein the amino acid sequence of VH (X) (where X is PD-1) consists of an amino acid sequence set forth in SEQ ID NO: 23 or 78, comprises the amino acid sequence, or has at least 90% identity with the amino acid sequence, and
   wherein the amino acid sequence of VL (X) (where X is PD-1) consists of an amino acid sequence set forth in SEQ ID NO: 28, 79, or 80, comprises the amino acid sequence, or has at least 90% identity with the amino acid sequence.
[15] The fusion protein according to [3], wherein the amino acid sequence of VH (X) (where X is PD-L1) consists of an amino acid sequence set forth in SEQ ID NO: 46 or 81, comprises the amino acid sequence, or has at least 90% identity with the amino acid sequence, and
   wherein the amino acid sequence of VL (X) (where X is PD-L1) consists of an amino acid sequence set forth in SEQ ID NO: 51, comprises the amino acid sequence, or has at least 90% identity with the amino acid sequence.
[16] The fusion protein according to [3], wherein the amino acid sequence of VH (X) (where X is PD-L1) consists of an amino acid sequence set forth in SEQ ID NO: 57, 109, 82, 83, or 84, comprises the amino acid sequence, or has at least 90% identity with the amino acid sequence, and
   wherein the amino acid sequence of VL (X) (where X is PD-L1) consists of an amino acid sequence set forth in SEQ ID NO: 61, 85, 86, or 87, comprises the amino acid sequence, or has at least 90% identity with the amino acid sequence.
[17] The fusion protein according to [3], wherein the amino acid sequence of VH (X) (where X is LAG3) consists of an amino acid sequence set forth in SEQ ID NO: 36, comprises the amino acid sequence, or has at least 90% identity with the amino acid sequence, and
   wherein the amino acid sequence of VL (X) (where X is LAG3) consists of an amino acid sequence set forth in SEQ ID NO: 40, comprises the amino acid sequence, or has at least 90% identity with the amino acid sequence.
[18] The fusion protein according to [1] or [2], wherein the first chain consists of an amino acid sequence set forth in SEQ ID NO: 1, comprises the amino acid sequence, or has at least 90% identity with the amino acid sequence, and
   wherein the second chain consists of an amino acid sequence set forth in SEQ ID NO: 17, 34, 44, 55, or 107 comprises the amino acid sequence, or has at least 90% identity with the amino acid sequence.
[19] The fusion protein according to [3], wherein the first chain, VH (5T4)-CH1-L1-VH (CD3)-L2-VL (CD3), consists of an amino acid sequence set forth in SEQ ID NO: 1, comprises the amino acid sequence, or has at least 90% identity with the amino acid sequence, and
   wherein the second chain, VL (5T4)CL-L1-VH (X)-L2-VL (X), consists of an amino acid sequence set forth in SEQ ID NO: 17, 34, 44, 55, or 107 comprises the amino acid sequence, or has at least 90% identity with the amino acid sequence.
[20] The fusion protein according to any one of [1] to [19], which is a trispecific antibody against (a) 5T4, (b) CD3 receptor complex, and (c) PD-1, PD-L1, or LAG3.
[21] The fusion protein according to any one of [1] to [20], which has anti-tumor activity.
[22] The fusion protein according to [21], wherein the tumor expresses 5T4 in tumor cells or cancer cells.
[23] The fusion protein according to[21] or [22], wherein the tumor is at least one selected from the group consisting of human mesothelioma (e.g., pleural mesothelioma), human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.
[24] A polynucleotide encoding the fusion protein according to any one of [1] to [23].
[25] A vector comprising the polynucleotide according to [24].
[26] A transformant obtained by introducing the vector according to [25] into a host cell.
[27] A method for producing the fusion protein according to any one of [1] to [23], which comprises culturing the transformant according to [26] and collecting the fusion protein from the obtained culture.
[28] A pharmaceutical composition comprising the fusion protein according to any one of [1] to [23].
[29] The pharmaceutical composition according to [28], which is used for treating, preventing, or diagnosing a tumor.
[30] The pharmaceutical composition according to [29], wherein the tumor expresses 5T4 in tumor cells or cancer cells.
[31] The pharmaceutical composition according to[29] or [30], wherein the tumor is at least one selected from the group consisting of human mesothelioma (e.g., pleural mesothelioma), human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.
[32] A method for treating, preventing, or diagnosing a tumor, which comprises administering the pharmaceutical composition according to any one of [28] to [31] to a subject.
[33] The method according to [32], wherein the tumor expresses 5T4 in tumor cells or cancer cells.
[34] The method according to[32] or [33], wherein the tumor is at least one selected from the group consisting of human mesothelioma (e.g., pleural mesothelioma), human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.
[35] A kit for treating, preventing, or diagnosing a tumor, which comprises the fusion protein according to any one of [1] to [23].
[36] The kit according to [35], wherein the tumor expresses 5T4 in tumor cells or cancer cells.
[37] The kit according to[35] or [36], wherein the tumor is at least one selected from the group consisting of human mesothelioma (e.g., pleural mesothelioma), human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.

### Advantageous Effects of Invention

According to the present invention, it is possible to provide a fusion protein or the like as a multispecific tribody whose anti-tumor efficacy is significantly increased as compared with the fusion protein Tb535H which is a conventional bispecific tribody. The fusion protein according to the present invention is a trispecific tribody that not only has ability to bind to 5T4 and a CD3 receptor complex, but also has ability to bind to PD-1, PD-L1 or LAG3 (that is, it also has an immune checkpoint inhibitory activity), and can exert excellent anti-tumor efficacy.

### Brief Description of Drawings

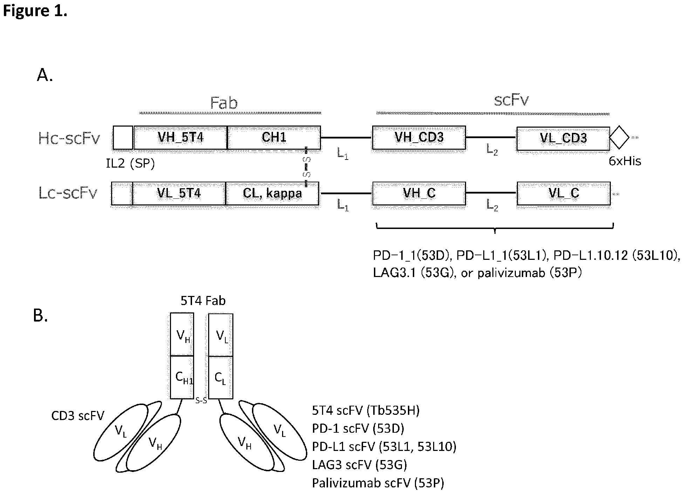

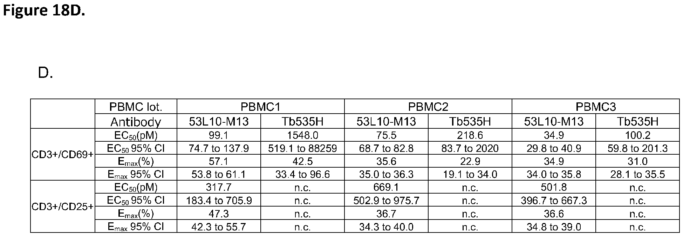

[Figure 1] Figure 1 is a schematic diagram of the structure of a tribody.
   A. Scheme of constructs for 53X tribody molecule. SP: human interleukin (IL)-2 signal peptide; VH_5T4, VL_5T4: amino acids sequences coding for the immunoglobulin heavy and light chain variable regions from Tb535H with specificity for human 5T4; CH1, CL: amino acids sequence coding for the human immunoglobulin heavy chain constant region 1 and the κ light chain constant region, respectively; VH_CD3, VL_CD3: amino acids sequences coding for the variable heavy and light chain regions from humanized OKT3 building a scFV with specificity for human CD3; L1, L2: amino acids sequences with flexible linker (comprising GPGGGSPG (SEQ ID NO: 6) and GGGGSGGGGSGGGGS [i.e., (GGGS)₃] (SEQ ID NO: 106), respectively); VH_C, VL_C: amino acids sequences coding for the heavy and light chain variable regions from PD-1-1, PD-L1_1, 10.12, LAG3_1, or palivizumab building scFV with specificity for human PD-1, PD-L1 or LAG3, the scFV from palivizumab is for an isotype human antibody-derived scFV control; 6xHis: amino acids sequences coding for a hexahistidine tag.
   B. Scheme of assembled tribody proteins for Tb535H[(5T4)₂ X CD3], 53D[5T4 x CD3 x PD-1], 53L1[5T4 x CD3 x PD-L1], 53L10[5T4 x CD3 x PD-L1], 53G [5T4 x CD3 x LAG3] and 53P [5T4 x CD3 x isotype control], respectively.
[Figure 2] Figure 2 shows binding affinity of Tb535H and constructed novel 53X tribodies to recombinant 5T4 protein in ELISA.
   A. Binding curves by ELISA assay of tribodies (0-500 nM) to recombinant human 5T4 proteins.
   B. EC₅₀ (nM) value for 5T4 binding of each construct.
[Figure 3] Figure 3 shows binding affinity of Tb535H and constructed novel 53X tribodies to recombinant CD3 protein in ELISA.
   A. Binding curves by ELISA assay of tribodies (0-500 nM) to recombinant human CD3δ/ε heterodimer proteins.
   B. EC₅₀ (nM) value for CD3 binding of each tribody construct.
[Figure 4] Figure 4 shows binding affinity of indicated tribodies to recombinant PD-L1 or PD-1 proteins in ELISA.
   A. Binding curves by ELISA assay of the 53L1 tribody and the 53L10 tribody (0-500 nM) to recombinant human PD-L1 proteins.
   B. Binding curves by ELISA assay of the 53D tribody (0-500 nM) to recombinant human PD-1 proteins.
[Figure 5] Figure 5 shows T cell activation bioassay in the presence of 5T4-expressing target cells.
   A. Schematic representation of TCR/CD3 activation in the presence of 5T4-expressing cells by T cell activation bioassay (NFAT) (Promega, Cat#. J1621).
   B. Genetically engineered Jurkat T cell line which expresses a luciferase reporter driven by an NFAT-response element (NFAT-RE) ("TCR/CD3 effector cells") cells were incubated with increasing concentration of the indicated tribodies (shown in the figure) in the presence of CHO-K1-5T4 cells. After 4 hr incubation at 37°C, Bio-GloTM reagent was added, and luminescence was measured by a luminometer.
   C. EC₅₀ (nM) value for 5T4-mediated TCR/CD3 activation of each construct.

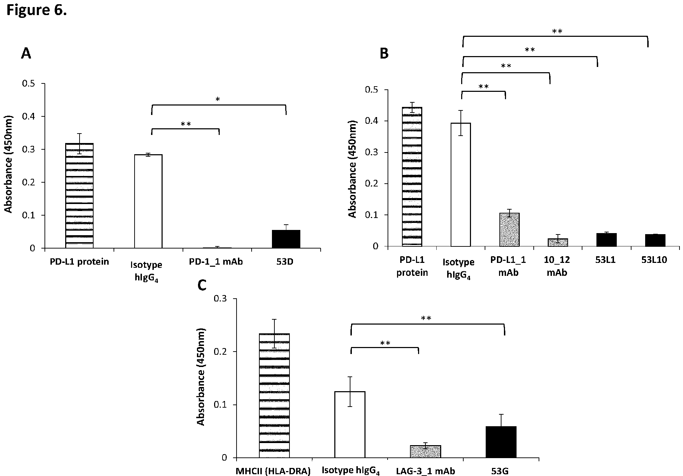

[Figure 6] Figure 6 shows antagonist effects of the 53X tribodies tested by Competitive ELISA assays.
   The ELISA assays were performed by measuring the binding of each biotin-labeled recombinant human PD-L1 or MHC II protein (striped bars) to PD-1 or LAG-3 immobilized proteins, respectively, in the absence or in the presence of the unlabelled competitive anti-PD-L1(53L1 and 51L10) (A), anti-PD-1 (53D) (B) or anti-LAG-3 (53G) (C) tribodies (black bars) or their respective parental mAbs (grey bars) used at saturating concentrations (5:1 for anti-PD-L1 or anti-PD-1, and 3:1 for anti-LAG-3). Human IgG4 isotype was used as a control (empty bars). Binding values were reported as the mean of at least three determinations obtained in three independent experiments. The values are shown as mean ± standard deviation (SD). p-values by student's t-test (two variables) are as follows: ** p < 0.01; * p < 0.05.

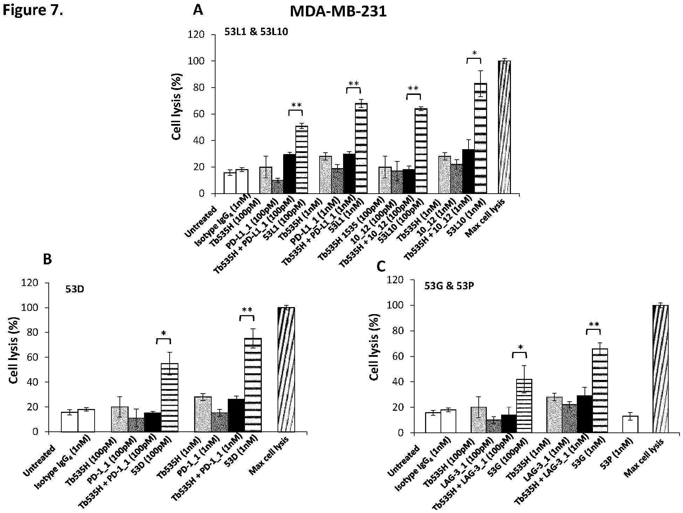

[Figure 7] Figure 7 shows cytotoxic activity of the novel tribodies or Tb535H administered alone or in combination with the parental mAbs on MDA-MB-231 human breast cancer cells co-cultured with hPBMCs.
   MDA-MB-231 human breast cancer cells were co-cultured with hPBMCs (Effector:Target cells ratio 5:1) and treated for 48 hours with 53L1 and 53L10 (A. striped bars), 53D (B. striped bars), or 53G (C: striped bars). Tb535H (light grey bars), parental immunomodulatory mAbs (dark grey bars) or their combinations (black bars) were also tested at the indicated concentrations. Untreated co-cultures or tribody 53P (C: empty bars) were used as negative controls. Cell lysis was measured by detecting LDH release, as described in "Materials and Methods" described later. Results are shown as mean ± SD. The following are p-values by student's t-test (two variables): *** p < 0,001; ** p < 0.01; * p < 0.05.

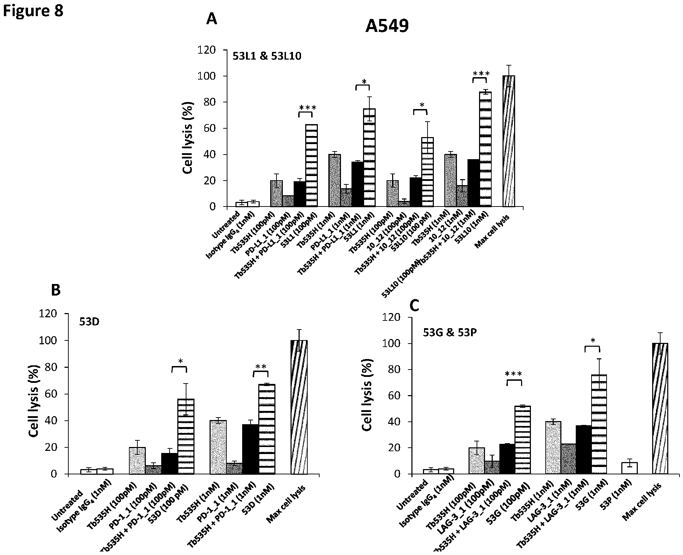

[Figure 8] Figure 8 shows cytotoxic activity of the novel tribodies or Tb535H administered alone or in combination with the parental mAbs on A549 human lung cancer cells co-cultured with hPBMCs.
   A549 human lung cancer cells were co-cultured with hPBMCs (Effector:Target cells ratio 5: 1) and treated for 48 hours with 53L1 and 53L10 (A. striped bars), 53D (B. striped bars), or 53G (C: striped bars). Tb535H (light grey bars), parental immunomodulatory mAbs (dark grey bars) or their combinations (black bars) were also tested at the indicated concentrations. Untreated co-cultures or tribody 53P (C: empty bars) were used as negative controls. Cell lysis was measured by detecting LDH release, as described in "Materials and Methods" described later. Results are shown as mean ± SD. The following are p-values by student's t-test (two variables): *** p < 0,001; ** p < 0.01; * p < 0.05.

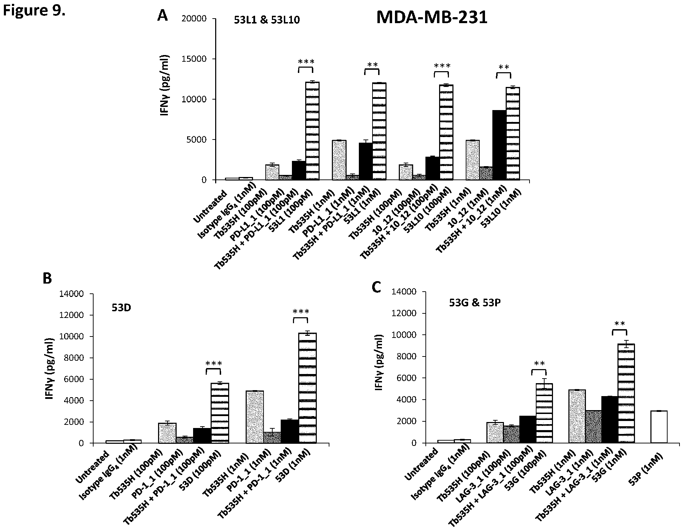

[Figure 9] Figure 9 shows effects of the novel tribodies or Tb535H administered alone or in combination with the parental mAbs on the secretion of IFN-γ by co-cultures of MDA-MB-231 human breast cancer cells with hPBMCs.
   The secretion of IFN-γ was measured by ELISA on supernatants of co-cultures of MDA-MB-231 human breast cancer cells with hPBMCs treated for 48 hours with 53L1 and 53L10 (A. striped bars), 53D (B. striped bars), or 53G (C: striped bars). Tb535H (light grey bars), parental immunomodulatory mAbs (dark grey bars) or their combinations (black bars) were also tested at the indicated concentrations. Untreated co-cultures or tribody 53P (C: empty bars) were used as negative controls. Results are shown as mean ± SD. The following are p-values by student's t-test (two variables): *** p < 0,001; ** p < 0.01; * p < 0.05.

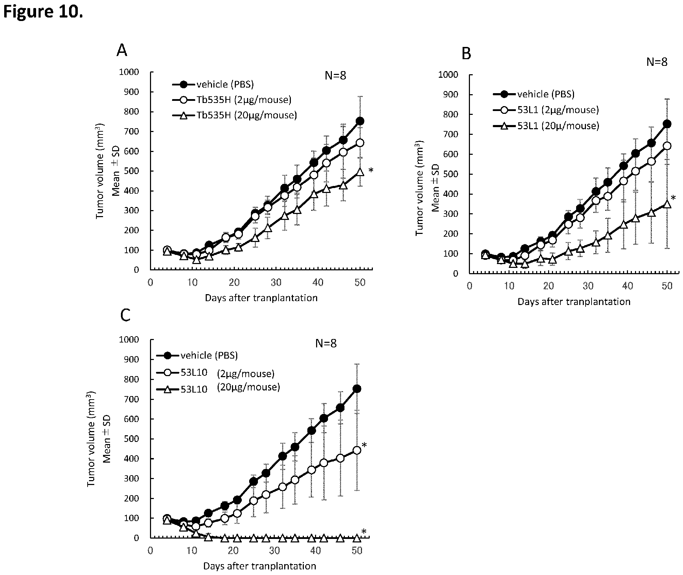

[Figure 10] Figure 10 shows *in vivo* anti-tumor efficacy of Tb535H and the novel tribodies 53L1 and 53L10.
   Tumor growth curves of A549 subcutaneous tumors in the presence of human PBMCs by treatment with (A) Tb535H, (B) 53L1, and (C) 53L10: ●: vehicle (PBS) treatment; ○: 2 µg/mouse treatment; △: 20 µg/mouse treatment. Five x 10⁶ cells of A549 human lung cancer cells were subcutaneously transplanted into right flank of NOD-scid mouse together with equal number of human PBMCs (hPBMCs). hPBMCs were stimulated with Dynabeads Human T-Activator CD3/CD28 (Veritas) for 4 days before transplantation. Intravenous treatment of indicated test article at the indicated dosage were performed at days 0, 2, 4, 6, 8, and 10 (total 6 treatments) after the cell transplantation. Tumor volume was expressed as mean ± standard deviation (SD). The significance test was performed by Dunnett's test (vs vehicle treatment group: * p < 0.05.

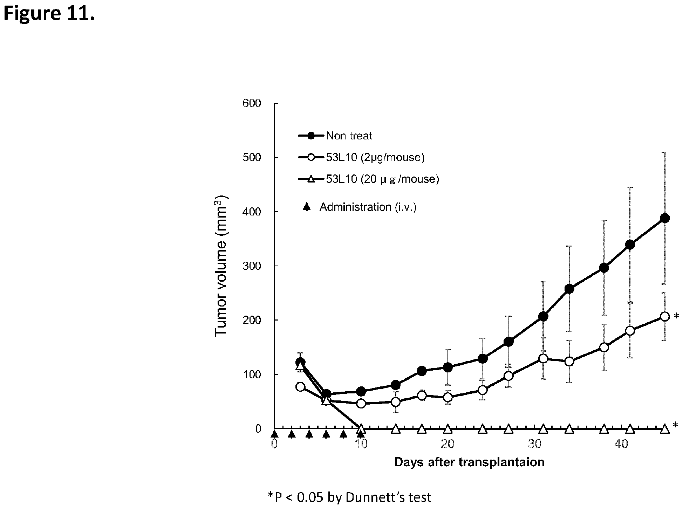

[Figure 11] Figure 11 shows tumor regression activity of the 53L10 tribody in different experimental sets:
   Tumor regrassion activity of 53L10 tribody in A549 xneograft model. Preparation of A549 cells and hPBMCs, subcutenous transplantation of mixed cells of A549 and hPBMCs were perfomed as the same as figure 10. 53L10 at the dosage of 2 µg/mouse (○) or 20 µg/mouse (△) were treated intravenously on day 0, 2, 4, 6, 8 and 10. Tumor growth in no treatment group (●) was used as a control group. Tumor volume was expressed as mean ± standard deviation (SD). The significance test was performed by Dunnett's test (vs no treatment group: * p < 0.05.

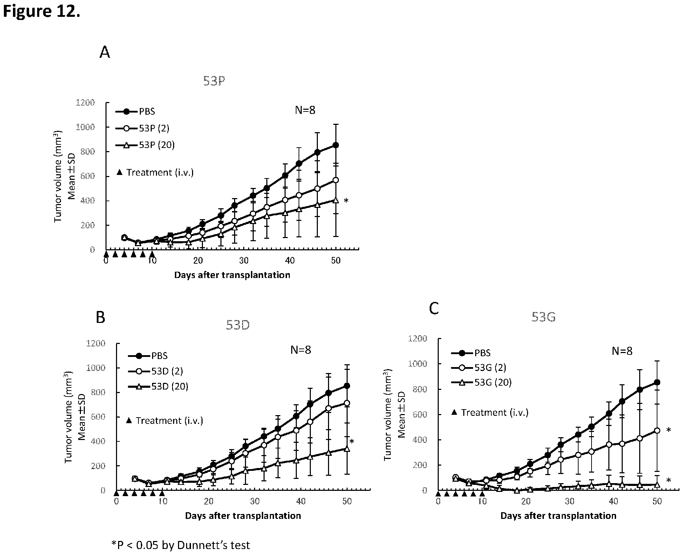

[Figure 12] Figure 12 shows *in vivo* anti-tumor efficacy of 53D and 53G compared to 53P. Tumor growth curves of A549 subcutaneous tumors in the presence of human PBMCs by treatment with (A) 53P (negative control), (B) 53D, and (C) 53G: ●: vehicle (PBS) treatment; ○: 2 µg/mouse treatment; △: 20 µg/mouse treatment. Tumor volume was expressed as mean ± standard deviation (SD). The significance test was performed by Dunnett's test (vs solvent treatment group: * p < 0.05.

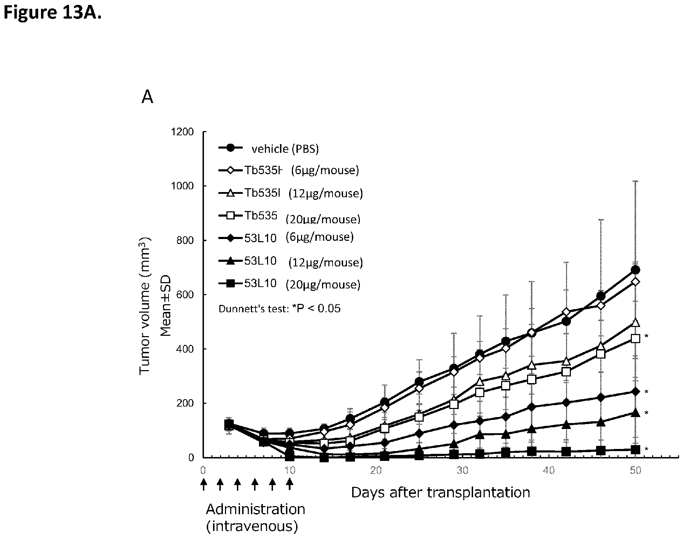

[Figure 13A] Figure 13A shows *in vivo* anti-tumor efficacy of Tb535H, administration of Tb535H in combination with pembrolizumab, and the novel tribody 53L10.
   A. Tumor growth curves of A549 subcutaneous tumors in the presence of human PBMCs by treatment with Tb535H or the novel tribody 53L10.
   B. Tumor growth curves of A549 subcutaneous tumors in the presence of human PBMCs by treatment with Tb535H in combination with pembrolizumab or the novel tribody 53L10.

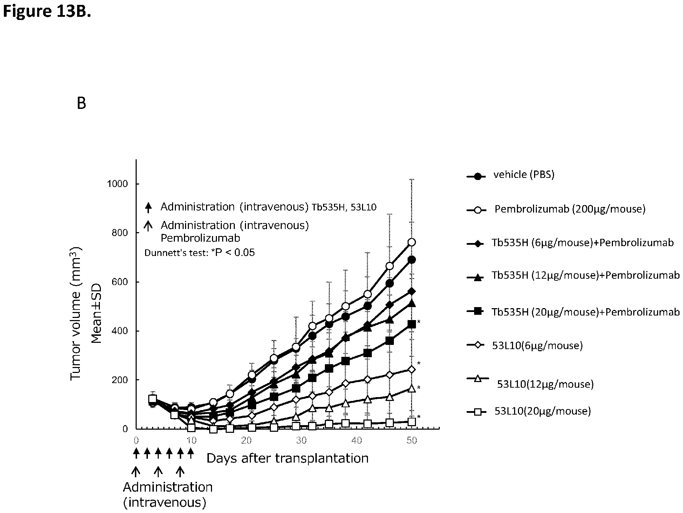

[Figure 13B] Figure 13B refers to the explanation in Figure 13A above.

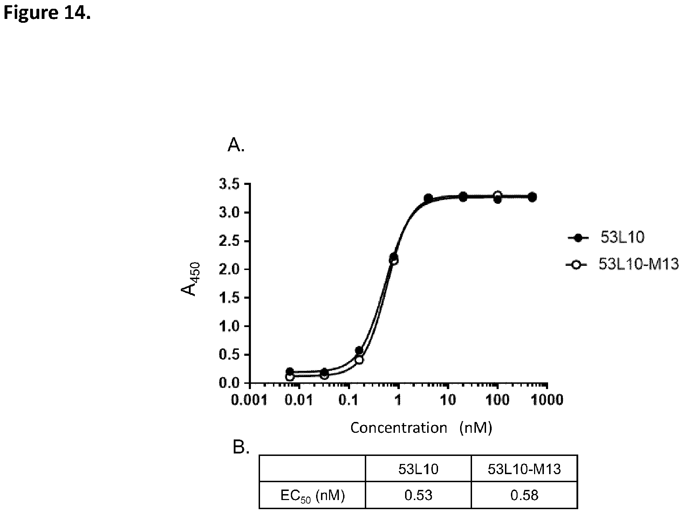

[Figure 14] Figure 14 shows binding affinity of 53L10 and 53L10-M13 to recombinant 5T4 protein in ELISA (N=2).
   A. Binding curves by ELISA assay of 53L10 and 53L10-M13 (0-500 nM) to recombinant human 5T4 protein.
   B. EC₅₀ (nM) value for 5T4 binding of each construct.

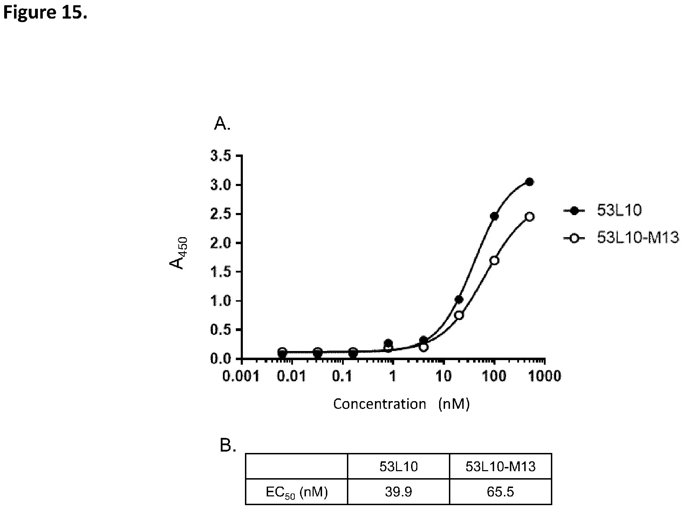

[Figure 15] Figure 15 shows binding affinity of 53L10 and 53L10-M13 to recombinant CD3 protein in ELISA (N=2).
   A. Binding curves by ELISA assay of 53L10 and 53L10-M13 (0-500 nM) to recombinant human CD3δ/ε heterodimer protein.
   B. EC₅₀ (nM) value for CD3 binding of each construct.

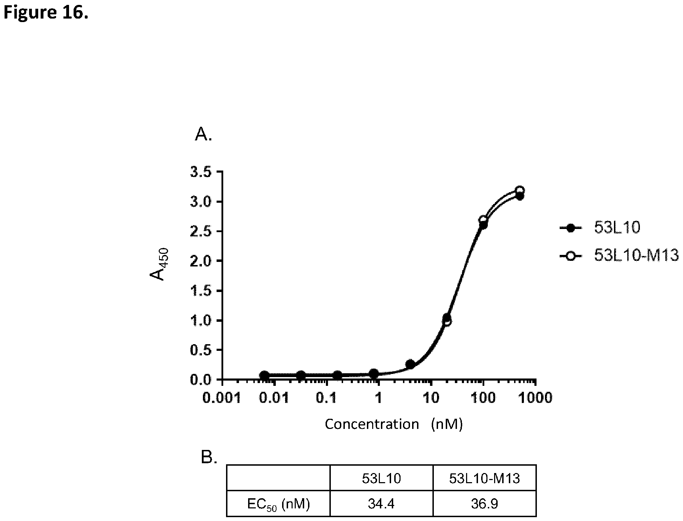

[Figure 16] Figure 16 shows binding affinity of 53L10 and 53L10-M13 to recombinant PD-L1 protein in ELISA (N=2).
   A. Binding curves by ELISA assay of 53L10 and 53L10-M13 (0-500 nM) to recombinant human PD-L1 protein.
   B. EC₅₀ (nM) value for PD-L1 binding of each construct.

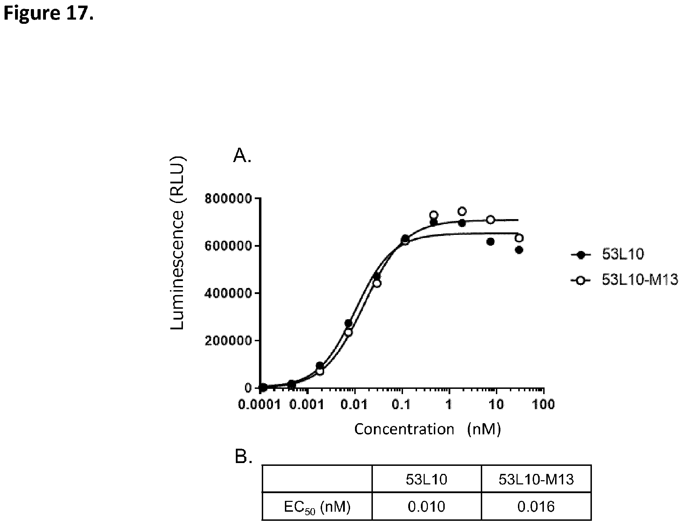

[Figure 17] Figure 17 shows T cell activation bioassay in the presence of 5T4-expressing target cells (N=2).
   A. Genetically engineered Jurkat T cell line which expresses a luciferase reporter driven by an NFAT-response element (NFAT-RE) ("TCR/CD3 effector cells") were incubated with increasing concentration of the indicated tribodies (shown in the figure) in the presence of CHO-K1-5T4 cells. After 6 hr incubation at 37°C, Bio-GloTM reagent was added, and luminescence was measured by a luminometer.
   B. EC₅₀ (nM) value for 5T4-mediated TCR/CD3 activation of each construct.

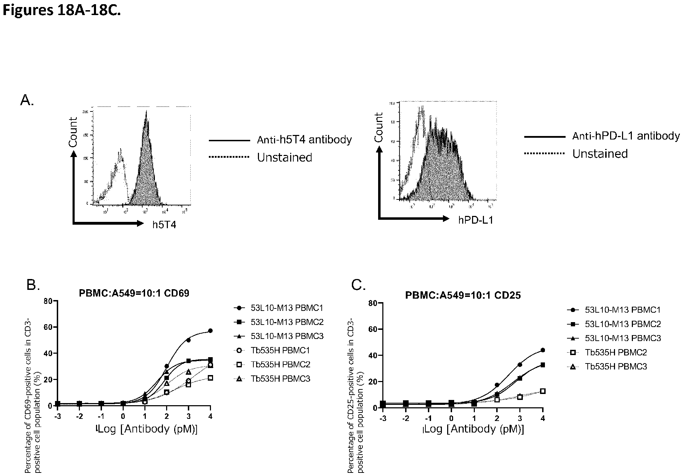

[Figures 18A-18C] Figures 18A-18C show measurement tests of T cell activation markers (CD25, CD69) for 53L10-M13 and Tb535H in the presence of h5T4- and hPD-L1-expressing target cancer cells (A549 human lung cancer cells).
   A. The expression of target molecules (h5T4, hPD-L1) on A549 human lung cancer cells was confirmed by flow cytometry.
   B. Percentage of CD69-positive cells to CD3-positive cells measured by flow cytometer.
   C. Percentage of CD25-positive cells to CD3-positive cells measured by flow cytometer.
   D. EC₅₀ (pM) and Eₘₐₓ (%) values of T cell activation of each construct evaluated with each T cell activation marker and their 95% confidence intervals (CIs). n.c. stands for "not calculated."
[Figure 18D] Figure 18D refers to the explanation in Figures 18A-18C above.

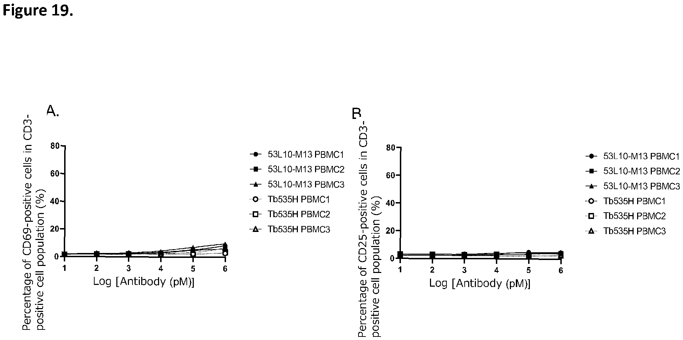

[Figure 19] Figure 19 shows measurement tests of T cell activation markers (CD25, CD69) for 53L10-M13 and Tb535H in the absence of the target cancer cell line.
   A. Percentage of CD69-positive cells to CD3-positive cells measured by flow cytometer.
   B. Percentage of CD25-positive cells to CD3-positive cells measured by flow cytometer.

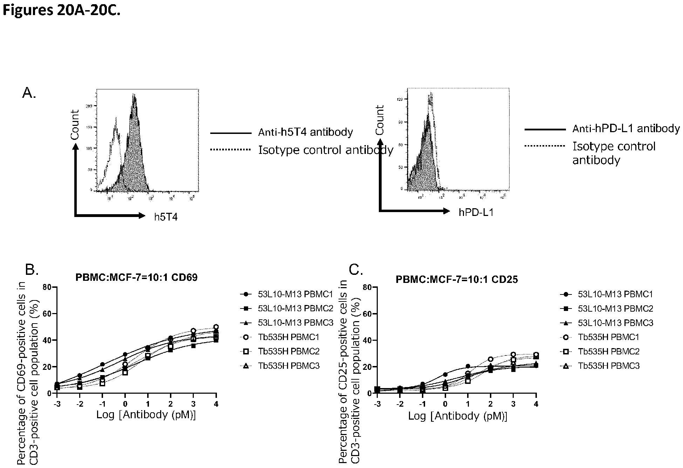

[Figures 20A-20C] Figures 20A-20C show measurement tests of T cell activation markers (CD25, CD69) for 53L10-M13 and Tb535H in the presence of h5T4-expressing target cancer cells (MCF-7 human breast cancer cells).
   A. The expression of target molecules (h5T4, hPD-L1) on MCF-7 human breast cancer cells was confirmed by flow cytometry.
   B. Percentage of CD69-positive cells to CD3-positive cells measured by flow cytometer.
   C. Percentage of CD25-positive cells to CD3-positive cells measured by flow cytometer.
   D. EC₅₀ (pM) and Eₘₐₓ (%) values of T cell activation of each construct evaluated with each T cell activation marker and their 95% confidence intervals.

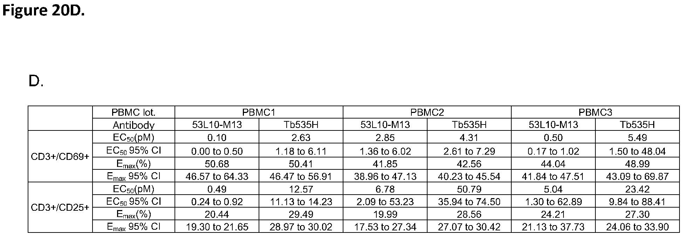

[Figure 20D] Figure 20D refers to the explanation in Figures 20A-20C above.

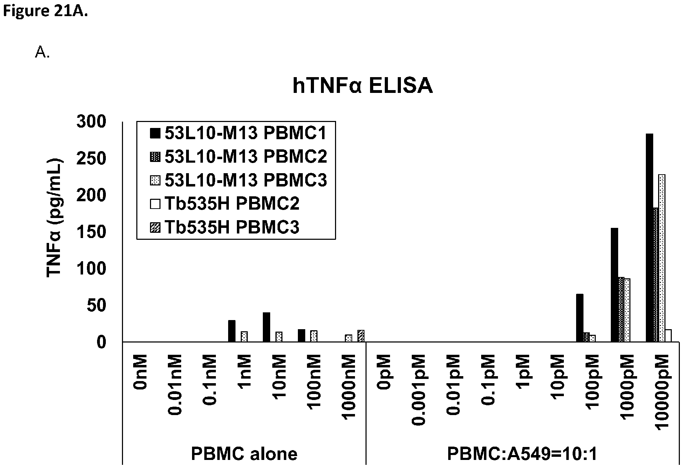

[Figure 21A] Figure 21A shows the concentration of free cytokines in the culture supernatant in a T cell activation test.
   A. hTNFα concentration measured by ELISA.
   B. hIL-2 concentration measured by ELISA.
   C. hIL-6 concentration measured by ELISA.
   D. Human interferon γ (hIFNγ) concentration measured by ELISA.

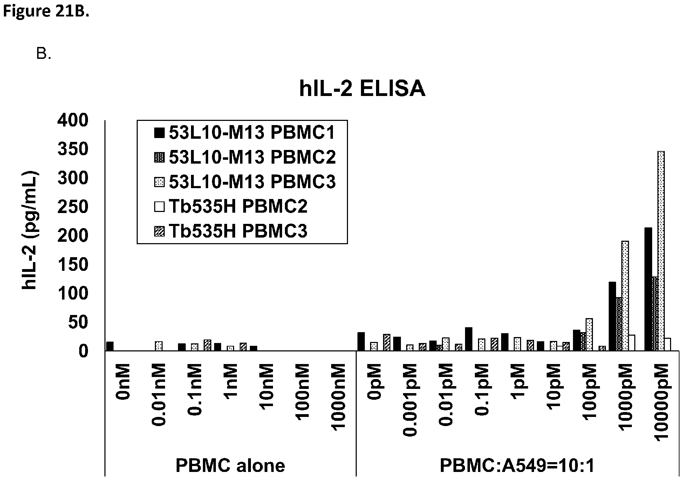

[Figure 21B] Figure 21B refers to the explanation in Figure 21A above.

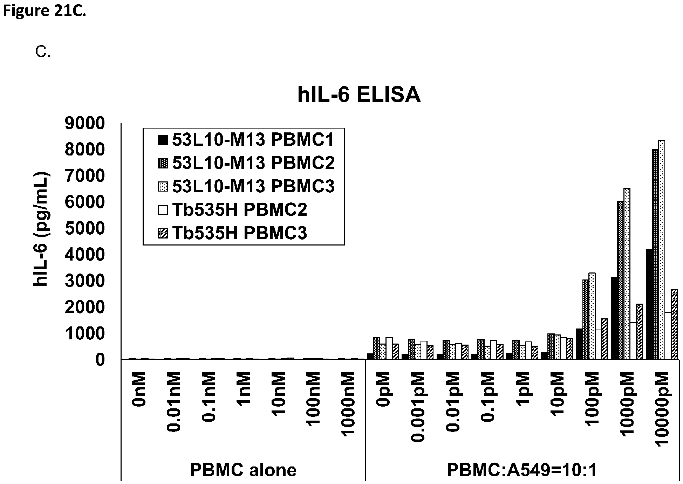

[Figure 21C] Figure 21C refers to the explanation in Figure 21A above.

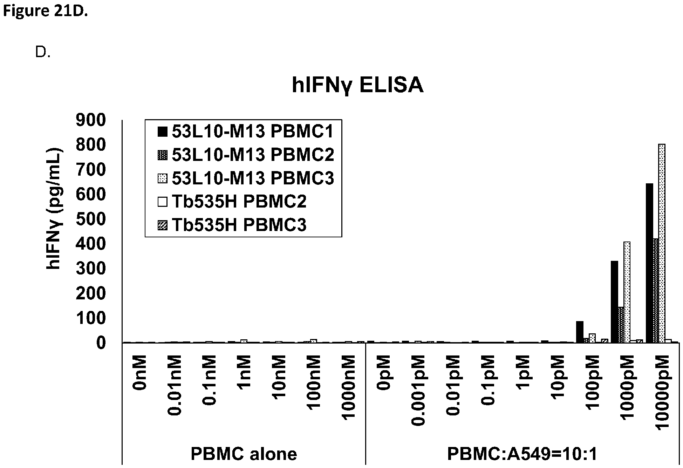

[Figure 21D] Figure 21D refers to the explanation in Figure 21A above.

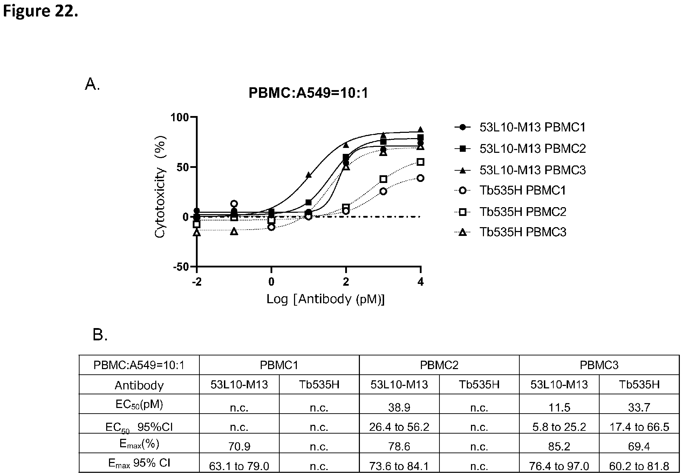

[Figure 22] Figure 22 shows cytotoxicity when hPBMC (without activation treatment) and 53L10-M13 or Tb535H were added to h5T4- and hPD-L1-expressing target cancer cells (A549 human lung cancer cells).
   A. Cytotoxicity measured by flow cytometer.
   B. EC₅₀ (pM) and Eₘₐₓ (%) values of cytotoxicity of each construct and their 95% confidence intervals.

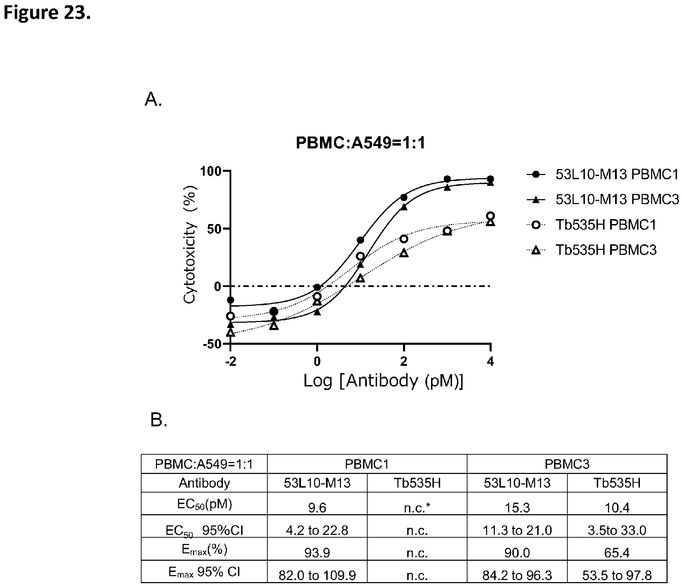

[Figure 23] Figure 23 shows cytotoxicity when activated hPBMC and 53L10-M13 or Tb535H were added to h5T4- and hPD-L1-expressing target cancer cells (A549 human lung cancer cells).
   A. Cytotoxicity measured by flow cytometer.
   B. EC₅₀ (pM) and Eₘₐₓ (%) values of cytotoxicity of each construct and their 95% confidence intervals.

### Description of Embodiments

Hereinafter, the present invention will be described in detail. The scope of the present invention is not limited to these explanations, and other than the following examples, the scope of the present invention can be appropriately modified and implemented as long as the gist of the present invention is not impaired.

This description incorporates the entirety of Japanese Patent Application No. 2022-106054 (filed on June 30, 2022), which is the basis of the priority claim of the present application. In addition, all publications cited herein, such as prior art documents, and publications, patent gazettes and other patent literature are incorporated herein by reference.

### 1. Overview of the Present Invention

As the fusion protein according to the present invention, a novel trispecific multifunctional tribody (also referred to as a trispecific antibody; 53X tribody) was constructed. In this invention, we generated novel tri-specific and multi-functional tribodies (53X tribodies). Novel tri-specific tribodies in this invention are composed of 5T4 binding Fab arm and CD3 binding scFv, but differently from the parental Tb535H the remaining scFv is derived from an antibody specific for immune check points, such as PD-1, PD-L1 or LAG-3 (see, for example, Patent Literature 2 and Non Patent Literature 10 above). Compared with parental Tb535H, which is a bi-specific T cell engager targeting 5T4, the novel 53X tribodies retained similar binding properties of the parental Tb535H tribody, but showed enhanced anti-tumor potency by incorporating checkpoint inhibition in a single molecule in addition to 5T4-mediated TCR/CD3 activation. In particular, the novel tribody called 53L10, a tri-specific T cell engager targeting 5T4, CD3 and PD-L1 immune checkpoint , showed the most promising anti-tumor efficacy *in vitro* and *in vivo.*

### 2. Fusion Protein (53X tribody)

The fusion protein according to the present invention is a fusion protein comprising two different first and second chains,
wherein the first chain comprises two antibody variable regions VH1 and VH2, one antibody variable region VL2, and one antibody constant region CH1 or CL, and
the second chain comprises two antibody variable regions VL1 and VL3, one antibody variable region VH3, and one antibody constant region CL or CH1.

Here, the "antibody variable region" is a motif of a polypeptide constituting an antibody molecule capable of specifically binding or interacting with a given antigen (e.g., a protein) and especially an epitope in the antigen. Each of the above VH1 to VH3 can be said to be a polypeptide consisting of a heavy chain variable region in an antibody molecule or a polypeptide containing the variable region, and each of the above VL1 to VL3 can be said to be a polypeptide consisting of a light chain variable region in an antibody molecule or a polypeptide containing the variable region. Then, VH1 and VL1, VH2 and VL2, and VH3 and VL3 are combined with each other to form a binding domain to a given antigen. In the present invention, these are referred to as "combinatorial VH/VL binding domains", and the combinations are referred to as "VH1/VL1 binding domain," "VH2/VL2 binding domain," and "VH3/VL3 binding domain." In addition, the "antibody constant region" is a polypeptide motif that constitutes an antibody molecule that is not directly involved in antigen binding but exhibits various effector functions such as antibody dependence, cell-mediated cellular cytotoxicity, and complement activation.

In the fusion protein according to the present invention, the first and second chains contain a heterodimeric interaction therebetween. The heterodimer interaction may be contained between CH1 of one chain and the CL domain of the other chain, or between VH1 and VL1, but of one chain is preferably contained between CH1 of one chain and the CL domain of the other chain. Due to such an interaction, the fusion protein according to the present invention is preferably a heterodimer protein of the first chain and the second chain. The heterodimer interaction is not limited, but in general, the interaction for heterodimerization conventionally known in Fab is preferable. Fab is a heterodimer between an Fd chain containing VH and CH1 antibody domains and an L chain containing VL and CL antibody domains, and for this heterodimerization, an interaction between VH1 and VL1 and/or between CH1 and CL (e.g., a disulfide bond) is formed. For the interaction, for example, Patent Literature 3 above can be referred to.

The fusion protein according to the present invention forms the above-described three different combinatorial VH/VL binding domains, that is, a VH1/VL1 binding domain, a VH2/VL2 binding domain, and a VH3/VL3 binding domain in the protein. These three binding domains have the abilities to bind to different substances (antigen proteins). Specifically, they are characterized by having any of the binding abilities selected from the following (i) to (iii):
(i) ability to bind to 5T4 (tumor-related antigen (TAA));
(ii) ability to bind to a CD3 receptor complex; and
(iii) ability to bind to PD-1 (programmed death receptor-1), PD-L1 (programmed cell death 1- ligand 1), or LAG3(lymphocyte activation gene 3: CD223).

A combinatorial VH/VL binding domain (or VH and VL themselves) habing the ability to bind to 5T4 (tumor-related antigen (TAA)) can be appropriately constructed or acquired by, for example, obtaining or preparing 5T4 as an antigen and preparing a polyclonal or monoclonal antibody against the antigen, and then preparing a desired fragment (VH, VL) derived from the antibody. Various combinatorial VH/VL binding domains (or VH and VL themselves) having the ability to bind to a CD3 receptor complex, ability to bind to PD-1, ability to bind to PD-L1, and ability to bind to LAG3 can be appropriately constructed and acquired by the same method. For details and exemplary explanations of preparation of antigens, preparation of polyclonal or monoclonal antibodies, preparation of recombinant antibodies (e.g., chimeric antibodies, humanized antibodies, and human antibodies (fully human antibodies)), and preparation of antibody fragments, for example, the descriptions in WO 2014/054820 and WO 2013/077458 can be appropriately referred to and applied.

The information on the amino acid sequence of human 5T4 (SEQ ID NO: 96) is disclosed as "Primary (citable) accession number:Q13641" on the Uniprot website(https://www.uniprot.org/(the same applies below)).

The information on the amino acid sequence (SEQ ID NO: 97) of the CD3 ε subunit of the human CD3 receptor complex is disclosed as, for example, "Primary (citable) accession number:P07766"on the Uniprot website, the information on the amino acid sequence (SEQ ID NO: 98) of the CD3 δ subunit (Isoform 1) is disclosed as, for example, "Primary (citable) accession number:P04234-1" on the Uniprot website, and the information on the amino acid sequence (SEQ ID NO: 99) of the CD3 δ subunit (Isoform 2) is disclosed as, for example, "Primary (citable) accession number:P04234-2" on the Uniprot website.

The information on the amino acid sequence of human PD-1 (SEQ ID NO: 100) is disclosed as "Primary (citable) accession number:Q15116" on the Uniprot website.

The information on the amino acid sequence (SEQ ID NO: 101) of human PD-L1 (Isoform 1) is disclosed as, for example, "Primary (citable) accession number:Q9NZQ7-1" on the Uniprot website, the information on the amino acid sequence (SEQ ID NO: 102) of human PD-L1 (Isoform 2) is disclosed as, for example, "Primary (citable) accession number:Q9NZQ7-2" on the Uniprot website, and the information on the amino acid sequence (SEQ ID NO: 103) of human PD-L1 (Isoform 3) is disclosed as, for example, "Primary (citable) accession number:Q9NZQ7-3" on the Uniprot website.

The information on the amino acid sequence (SEQ ID NO: 104) of human LAG3 (Isoform 1) is discloses as, for example, "Primary (citable) accession number:P18627-1" on the Uniprot website, and the information on the amino acid sequence (SEQ ID NO: 105) of human LAG3 (Isoform 2) is disclosed as, for example, "Primary (citable) accession number:P18627-2" on the Uniprot website.These sequence information can be appropriately used in the preparation of antigens and the production or preparation of antibodies and antibody fragments.

In the fusion protein according to the present invention, VH1, VH2, and VH3 have different complementarity determining regions (CDRs), which are CDR 1, CDR2, and CDR3, respectively. The amino acid sequences of CDRs 1, 2 and 3 that can be adopted in VH1, VH2, and VH3 are not limited, but consist respectively of:
amino acid sequences set forth in SEQ ID NOS: 3, 4, and 5;
amino acid sequences set forth in SEQ ID NOS: 9, 10, and 11;
amino acid sequences set forth in SEQ ID NOS: 24, 25, and 26 or 27 (the amino acid sequence of SEQ ID NO: 27 can be preferably adopted in place of the amino acid sequence of SEQ ID NO: 26);
amino acid sequences set forth in SEQ ID NOS: 37, 38, and 39;
amino acid sequences set forth in SEQ ID NOS: 47, 48 or 49, and 50; or
amino acid sequences set forth in SEQ ID NOS: 58, 59 or 49, and 60
as preferred examples.

Similarly, in the fusion protein according to the present invention, VL1, VL2, and VL3 have different complementarity determining regions (CDRs), which are CDR 1, CDR2, and CDR3, respectively. The amino acid sequences of CDRs 1, 2 and 3 that can be adopted in VL1, VL2, and VL3 are not limited, but consist respectively of:
amino acid sequences set forth in SEQ ID NOS: 19, 20, and 21;
amino acid sequences set forth in SEQ ID NOS: 14, 15, and 16;
amino acid sequences set forth in SEQ ID NOS: 29, 30, and 31, 32, or 33 (the amino acid sequences of SEQ ID NOS: 32 and 33 can be preferably adopted in place of the amino acid sequence of SEQ ID NO: 31);
amino acid sequences set forth in SEQ ID NOS: 41, 42, and 43;
amino acid sequences set forth in SEQ ID NOS: 52, 53, and 54; or
amino acid sequences set forth in SEQ ID NOS: 62, 63, and 64
as preferred examples.

Further, the amino acid sequences of VH1, VH2, and VH3 in the fusion protein according to the present invention are different from each other. Preferred examples thereof include those consisting of the amino acid sequence set forth in SEQ ID NO: 88, 90, 8, 92, 23, 78, 46, 81, 57, 109, 82, 83, 84, or 36, those comprising the amino acid sequence, and those having at least 90% (which may be 95% or more, 96% or more, 97% or more, 98% or more, or 99% or more) identity with the amino acid sequence. Here, the amino acid of SEQ ID NO: 78 can be preferably adopted in place of the amino acid of SEQ ID NO: 23, the amino acid of SEQ ID NO: 81 can be preferably adopted in place of the amino acid of SEQ ID NO: 46, and the amino acids of SEQ ID NOS: 82 to 84 and 109 can be preferably adopted in place of the amino acids of SEQ ID NO: 57.

In addition, the amino acid sequences of VL1, VL2, and VL3 in the fusion protein according to the present invention are different from each other. Preferred examples thereof include those consisting of the amino acid sequence set forth in SEQ ID NO: 89, 91, 13, 93, 28, 79, 80, 51, 61, 85, 86, 87, or 40, those comprising the amino acid sequence, and those having at least 90% (which may be 95% or more, 96% or more, 97% or more, 98% or more, or 99% or more) identity with the amino acid sequence. Here, the amino acids of SEQ ID NO: 79 and 80 can be preferably adopted in place of the amino acid of SEQ ID NO: 28, and the amino acids of SEQ ID NOS: 85 to 87 can be preferably adopted in place of the amino acids of SEQ ID NO: 61.

Further, it is preferable that the first chain in the fusion protein according to the present invention is, for example, one consisting of the amino acid sequence set forth in SEQ ID NO: 1, one comprising the amino acid sequence, or one having at least 90% (which may be 95% or more, 96% or more, 97% or more, 98% or more, or 99% or more) identity with the amino acid sequence. In addition, it is preferable that the second chain in the fusion protein according to the present invention is, for example, one consisting of the amino acid sequence set forth in SEQ ID NO: 17, 34, 44, 55, or 107, one comprising the amino acid sequence, or one having at least 90% (which may be 95% or more, 96% or more, 97% or more, 98% or more, or 99% or more) identity with the amino acid sequence.

As another aspect of the fusion protein according to the present invention, for example, it is preferable that the combination of the two different first and second chains is one of the combinations shown in a) to l) below.
a) A first chain:
   VH (5T4)-CH1-L1-VH (CD3)-L2-VL (CD3) or
   VH (5T4)-CH1-L1-VL (CD3)-L2-VH (CD3) combined with a second chain:
      VL (5T4)-CL-L1-VH (X)-L2-VL (X) or
      VL (5T4)-CL-L1-VL (X)-L2-VH (X).
b) A first chain:
   VH (5T4)-CH1-L1-VH (X)-L2-VL (X) or
   VH (5T4)-CH1-L1-VL (X)-L2-VH (X) combined with a second chain:
      VL (5T4)-CL-L1-VH (CD3)-L2-VL (CD3) or
      VL (5T4)-CL-L1-VL (CD3)-L2-VH (CD3).
c) A first chain:
   VL (5T4)-CH1-L1-VH (CD3)-L2-VL (CD3) or
   VL (5T4)-CH1-L1-VL (CD3)-L2-VH (CD3) combined with a second chain:
      VH (5T4)-CL-L1-VH (X)-L2-VL (X) or
      VH (5T4)-CL-L1-VL (X)-L2-VH (X).
d) A first chain:
   VL (5T4)-CH1-L1-VH (X)-L2-VL (X) or
   VL (5T4)-CH1-L1-VL (X)-L2-VH (X) combined with a second chain:
      VH (5T4)-CL-L1-VH (CD3)-L2-VL (CD3) or
      VH (5T4)-CL-L1-VL (CD3)-L2-VH (CD3).
e) A first chain:
   VH (CD3)-CH1-L1-VH (5T4)-L2-VL (5T4) or
   VH (CD3)-CH1-L1-VL (5T4)-L2-VH (5T4) combined with a second chain:
      VL (CD3)-CL-L1-VH (X)-L2-VL (X) or
      VL (CD3)-CL-L1-VL (X)-L2-VH (X).
f) A first chain:
   VH (CD3)-CH1-L1-VH (X)-L2-VL (X) or
   VH (CD3)-CH1-L1-VL (X)-L2-VH (X) combined with a second chain:
      VL (CD3)-CL-L1-VH (5T4)-L2-VL (5T4) or
      VL (CD3)-CL-L1-VL (5T4)-L2-VH (5T4).
g) A first chain:
   VL (CD3)-CH1-L1-VH (5T4)-L2-VL (5T4) or
   VL (CD3)-CH1-L1-VL (5T4)-L2-VH (5T4) combined with a second chain:
      VH (CD3)-CL-L1-VH (X)-L2-VL (X) or
      VH (CD3)-CL-L1-VL (X)-L2-VH (X).
h) A first chain:
   VL (CD3)-CH1-L1-VH (X)-L2-VL (X) or
   VL (CD3)-CH1-L1-VL (X)-L2-VH (X) combined with a second chain:
      VH (CD3)-CL-L1-VH (5T4)-L2-VL (5T4) or
      VH (CD3)-CL-L1-VL (5T4)-L2-VH (5T4).
i) A first chain:
   VH (X)-CH1-L1-VH (5T4)-L2-VL (5T4) or
   VH (X)-CH1-L1-VL (5T4)-L2-VH (5T4) combined with a second chain:
      VL (X)-CL-L1-VH (CD3)-L2-VL (CD3) or
      VL (X)-CL-L1-VL (CD3)-L2-VH (CD3).
j) A first chain:
   VH (X)-CH1-L1-VH (CD3)-L2-VL (CD3) or
   VH (X)-CH1-L1-VL (CD3)-L2-VH (CD3) combined with a second chain:
      VL (X)-CL-L1-VH (5T4)-L2-VL (5T4) or
      VL (X)-CL-L1-VL (5T4)-L2-VH (5T4).
k) A first chain:
   VL (X)-CH1-L1-VH (5T4)-L2-VL (5T4) or
   VL (X)-CH1-L1-VL (5T4)-L2-VH (5T4) combined with a second chain:
      VH (X)-CL-L1-VH (CD3)-L2-VL (CD3) or
      VH (X)-CL-L1-VL (CD3)-L2-VH (CD3).
l) A first chain:
   VL (X)-CH1-L1-VH (CD3)-L2-VL (CD3) or
   VL (X)-CH1-L1-VL (CD3)-L2-VH (CD3) combined with a second chain:
      VH (X)-CL-L1-VH (5T4)-L2-VL (5T4) or
      VH (X)-CL-L1-VL (5T4)-L2-VH (5T4).

Here, in the first and/or second chains shown in a) to l) above,
VH and VL are antibody variable regions,
VH (5T4) and VL (5T4) are antibody variable regions for 5T4,
VH (CD3) and VL (CD3) are antibody variable regions for the CD3 receptor complex,
VH (X) and VL (X) are antibody variable regions for PD-1, PD-L1, or LAG3,
CH1 and CL are antibody constant regions, and
L1 and L2 are linkers.

More specifically, it is preferable that the amino acid sequences of complementarity determining regions (CDRs) 1, 2, and 3 in VH (5T4) consist respectively of amino acid sequences set forth in SEQ ID NOS: 3, 4, and 5, and the amino acid sequences of CDRs 1, 2, and 3 in VL(5T4) consist respectively of:
amino acid sequences set forth in SEQ ID NOS: 19, 20, and 21.

It is more preferable that the amino acid sequence of VH (5T4) is one consisting of the amino acid sequence set forth in SEQ ID NO: 88, or 90, one comprising the amino acid sequence, or one having at least 90% (which may be 95% or more, 96% or more, 97% or more, 98% or more, or 99% or more) identity with the amino acid sequence, and the amino acid sequence of VL (5T4) is one consisting of the amino acid sequence set forth in SEQ ID NO: 89 or 91, one comprising the amino acid sequence, or one having at least 90% (which may be 95% or more, 96% or more, 97% or more, 98% or more, or 99% or more) identity with the amino acid sequence.

More specifically, it is preferable that the amino acid sequences of complementarity determining regions (CDRs) 1, 2, and 9 in VH (CD3) consist respectively of amino acid sequences set forth in SEQ ID NOS: 3, 10, and 11, and
the amino acid sequences of CDRs 1, 2, and 3 in VL(CD3) consist respectively of: amino acid sequences set forth in SEQ ID NOS: 14, 15, and 16.

It is more preferable that the amino acid sequence of VH (CD3) is one consisting of the amino acid sequence set forth in SEQ ID NO: 8, or 92, one comprising the amino acid sequence, or one having at least 90% (which may be 95% or more, 96% or more, 97% or more, 98% or more, or 99% or more) identity with the amino acid sequence, and the amino acid sequence of VL (CD3) is one consisting of the amino acid sequence set forth in SEQ ID NO: 13 or 93, one comprising the amino acid sequence, or one having at least 90% (which may be 95% or more, 96% or more, 97% or more, 98% or more, or 99% or more) identity with the amino acid sequence.

More specifically, it is preferable that the amino acid sequences of complementarity determining regions (CDRs) 1, 2, and 3 in VH (X) (where X is PD-1) consist respectively of amino acid sequences set forth in SEQ ID NOS: 24, 25, and 26 or 27, and the amino acid sequences of CDRs 1, 2, and 3 in VL (X) (where X is PD-1) consist respectively of amino acid sequences set forth in SEQ ID NOS: 29, 30, and 31, 32, or 33.

It is more preferable that the amino acid sequence of VH (X) (where X is PD-1) is one consisting of the amino acid sequence set forth in SEQ ID NO: 23 or 78, one comprising the amino acid sequence, or one having at least 90% (which may be 95% or more, 96% or more, 97% or more, 98% or more, or 99% or more) identity with the amino acid sequence, and the amino acid sequence of VL (X) (where X is PD-1) is one consisting of the amino acid sequence set forth in SEQ ID NO: 28, 79, or 80, one comprising the amino acid sequence, or one having at least 90% (which may be 95% or more, 96% or more, 97% or more, 98% or more, or 99% or more) identity with the amino acid sequence.

More specifically, it is preferable that the amino acid sequences of complementarity determining regions (CDRs) 1, 2, and 3 in VH (X) (where X is PD-L1 (more specifically PD-L11)) consist respectively of amino acid sequences set forth in SEQ ID NOS: 47, 48 or 49, and 50, and the amino acid sequences of CDRs 1, 2, and 3 in VL (X) (where X is PD-L1 (more specifically PD-L11)) consist respectively of amino acid sequences set forth in SEQ ID NOS: 52, 53, and 54.

It is more preferable that the amino acid sequence of VH (X) (where X is PD-L1 (more specifically PD-L11)) is one consisting of the amino acid sequence set forth in SEQ ID NO: 46 or 81, one comprising the amino acid sequence, or one having at least 90% (which may be 95% or more, 96% or more, 97% or more, 98% or more, or 99% or more) identity with the amino acid sequence, and the amino acid sequence of VL (X) (where X is PD-L1 (more specifically PD-L11)) is one consisting of the amino acid sequence set forth in SEQ ID NO: 51, one comprising the amino acid sequence, or one having at least 90% (which may be 95% or more, 96% or more, 97% or more, 98% or more, or 99% or more) identity with the amino acid sequence.

More specifically, it is preferable that the amino acid sequences of complementarity determining regions (CDRs) 10.12, 2, and 3 in VH (X) (where X is PD-L1 (more specifically 10.12)) consist respectively of amino acid sequences set forth in SEQ ID NOS: 58, 59 or 49, and 60, and the amino acid sequences of CDRs 10.12, 2, and 3 in VL (X) (where X is PD-L1 (more specifically 10.12)) consist respectively of amino acid sequences set forth in SEQ ID NOS: 62, 63, and 64.

It is more preferable that the amino acid sequence of VH (X) (where X is PD-L1 (more specifically 10.12)) is one consisting of the amino acid sequence set forth in SEQ ID NO: 57, 109, 82, 83, or 84, one comprising the amino acid sequence, or one having at least 90% (which may be 95% or more, 96% or more, 97% or more, 98% or more, or 99% or more) identity with the amino acid sequence, and the amino acid sequence of VL (X) (where X is PD-L1 (more specifically 10.12)) is one consisting of the amino acid sequence set forth in SEQ ID NO: 61, 85, 86, or 87, one comprising the amino acid sequence, or one having at least 90% (which may be 95% or more, 96% or more, 97% or more, 98% or more, or 99% or more) identity with the amino acid sequence.

More specifically, it is preferable that the amino acid sequences of complementarity determining regions (CDRs) 1, 2, and 3 in VH (X) (where X is LAG3) consist respectively of amino acid sequences set forth in SEQ ID NOS: 37, 38, and 39, and the amino acid sequences of CDRs 1, 2, and 3 in VL (X) (where X is LAG3) consist respectively of amino acid sequences set forth in SEQ ID NOS: 41, 42, and 43.

It is more preferable that the amino acid sequence of VH (X) (where X is LAG3) is one consisting of the amino acid sequence set forth in SEQ ID NO: 36, one comprising the amino acid sequence, or one having at least 90% (which may be 95% or more, 96% or more, 97% or more, 98% or more, or 99% or more) identity with the amino acid sequence, and the amino acid sequence of VL (X) (where X is LAG3) is one consisting of the amino acid sequence set forth in SEQ ID NO: 40, one comprising the amino acid sequence, or one having at least 90% (which may be 95% or more, 96% or more, 97% or more, 98% or more, or 99% or more) identity with the amino acid.

More specifically, it is preferable that the amino acid sequence of CH1 that is an antibody constant region is one consisting of the amino acid sequence set forth in SEQ ID NO: 94, one comprising the amino acid sequence, or one having at least 90% (which may be 95% or more, 96% or more, 97% or more, 98% or more, or 99% or more) identity with the amino acid sequence.

It is preferable that the amino acid sequence of CL that is an antibody constant region is one consisting of the amino acid sequence set forth in SEQ ID NO: 95, one comprising the amino acid sequence, or one having at least 90% (which may be 95% or more, 96% or more, 97% or more, 98% or more, or 99% or more) identity with the amino acid sequence.

It is preferable that L1 that is a linker is one consisting of the amino acid sequence set forth in SEQ ID NO: 6, one comprising the amino acid sequence, or one having at least 90% (which may be 95% or more, 96% or more, 97% or more, 98% or more, or 99% or more) identity with the amino acid sequence.

It is preferable that L2 that is a linker is one consisting of the amino acid sequence set forth in SEQ ID NO: 12 or 106, one comprising the amino acid sequence, or one having at least 90% (which may be 95% or more, 96% or more, 97% or more, 98% or more, or 99% or more) identity with the amino acid sequence.

More specifically, for the fusion protein according to the present invention, it is more preferable that the first chain (VH (5T4) -CH1-L1-VH (CD3) -L2-VL (CD3)) is one consisting of the amino acid sequence set forth in SEQ ID NO: 1, one comprising the amino acid sequence, or one having at least 90% (which may be 95% or more, 96% or more, 97% or more, 98% or more, or 99% or more) identity with the amino acid sequence, and the second chain (VL(5T4)CL-L1-VH(X)-L2-VL(X)) is one consisting of the amino acid sequence set forth in SEQ ID NO: 17, 34, 44, 55, or 107 one comprising the amino acid sequence, or one having at least 90% (which may be 95% or more, 96% or more, 97% or more, 98% or more, or 99% or more) identity with the amino acid sequence.

For the fusion protein of the present invention included in the preferred embodiments, a table listing the full lengths of the H chain derivatives and various L chain derivatives, and the SEQ ID NOS of the amino acid sequences of the individual domains, regions, and linkers is shown below.

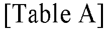

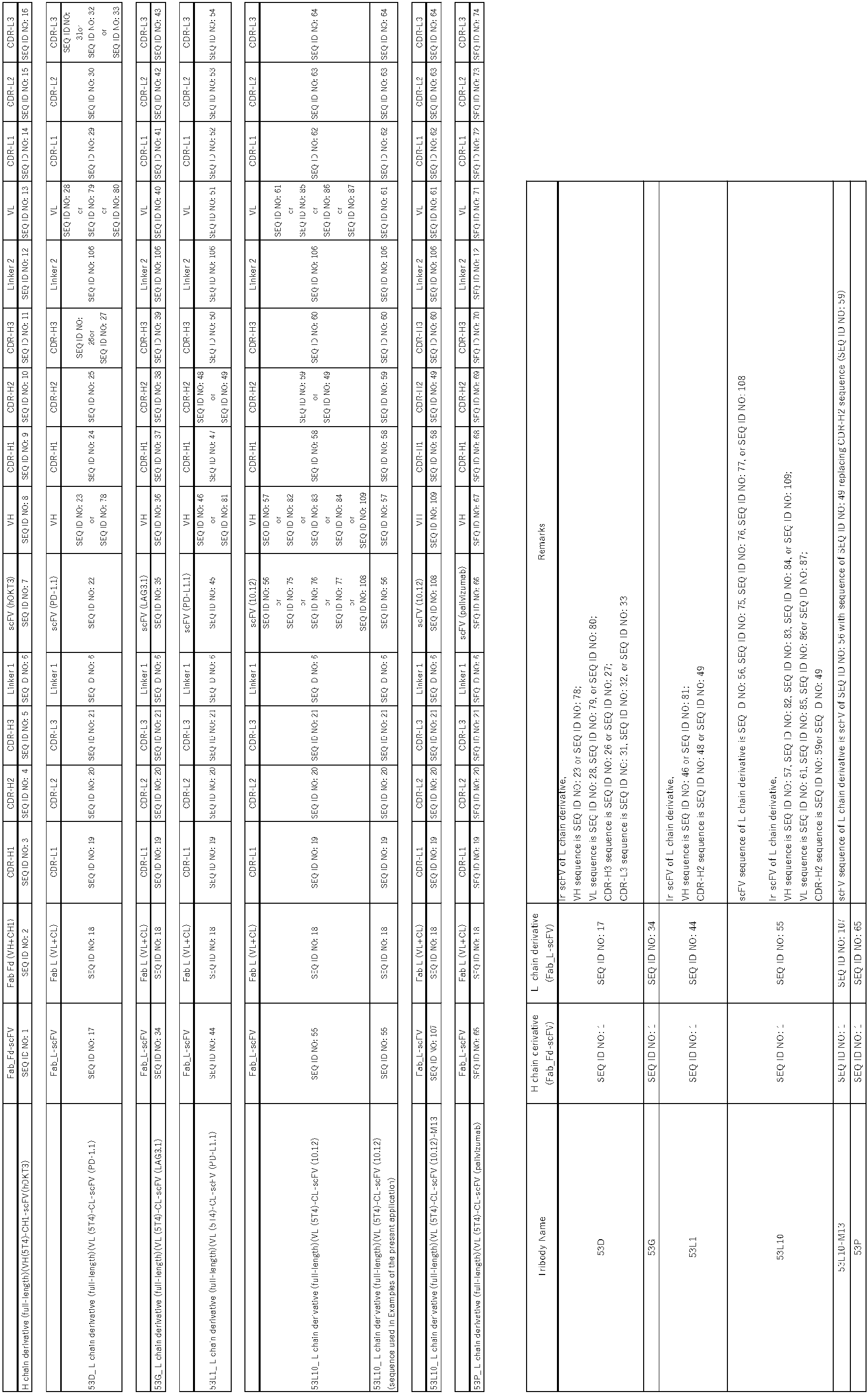

The fusion protein according to the present invention, which can be specifically explained as described above, is preferably: (a) 5T4, (b) a CD3 receptor complex on T cells, and (c) a trispecific antibody (tribody) against PD-1, PD-L1, or LAG3. Accordingly, the antibody has anti-tumor activity, and furthermore, the anti-tumor efficacy of the antibody is significantly increased as compared with the conventional Tb535H tribody and the combination therapy with the anti-PD-1 antibody and the anti-PD-L1 antibody. Among them, a trispecific antibody against (a) 5T4, (b) a CD3 receptor complex on T cells, and (c) PD-L1 is preferable in that it has excellent anti-tumor efficacy. Accordingly, the present invention also provides a fusion protein as a trispecific antibody that can be used in the treatment, prevention, or diagnosis of a tumor or a cancer.

Here, the tumor is not limited, and one expressing 5T4 in tumor cells or cancer cells is preferable. Specific examples thereof include at least one selected from the group consisting of human mesothelioma (e.g., pleural mesothelioma), human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor. The examples of these tumors are similarly applied in the following description as appropriate.

### 3. Polynucleotides, Recombinant Vectors, and Transformants

In the present invention, the polynucleotide (gene, DNA) encoding the fusion protein (53X tribody) according to the present invention described above can also be provided. Specifically, the polynucleotide is preferably a polynucleotide comprising a base sequence encoding each amino acid sequence shown as an example of the fusion protein according to the present invention described above. The polynucleotide of the present invention is not limited and it may consist of a polynucleotide encoding the fusion protein according to the present invention or may be a polynucleotide containing the polynucleotide in part and also known nucleotide sequences required for gene expression (e.g., a transcription promoter, an SD sequence, a Kozak sequence, and a terminator) and/or an additional sequence (or a tag sequence, for example, a 6×His sequence) that facilitates protein purification.

The polynucleotide encoding the fusion protein according to the present invention is not particularly limited in terms of codons corresponding to individual amino acids after translation and it may be a polynucleotide comprising nucleotide DNA indicating a codon (preferably a frequently used codon) commonly used in mammals such as humans after transcription or a polynucleotide comprising nucleotide DNA indicating a codon (preferably a frequently used codon) commonly used in microorganisms such as *Escherichia coli* and yeast, plants, and the like.

In the present invention, a recombinant vector containing the above-described polynucleotide of the present invention and a transformant containing the recombinant vector can also be provided.

If necessary, the polynucleotide (gene, DNA) to be incorporated into the expression vector used as the recombinant vector may be ligated upstream to a transcription promoter, an SD sequence (when the host is a prokaryotic cell), and a Kozak sequence (when the host is a eukaryotic cell), may be ligated downstream to a terminator, and additionally, may be ligated to an enhancer, a splicing signal, a poly A addition signal, a selectable marker, and the like. Each element necessary for gene expression such as the transcription promoter may be contained in the polynucleotide from the beginning. In a case in which such elements are originally contained in the expression vector, they may be used. The form of each element when used is not particularly limited.

As a method for incorporating the polynucleotide into the expression vector, various methods using known gene recombination techniques such as a method using a restriction enzyme and a method using a topoisomerase can be adopted. The expression vector is not limited as long as it can contain a polynucleotide (gene, DNA) encoding the fusion protein according to the present invention, such as plasmid DNA, bacteriophage DNA, retrotransposon DNA, retrovirus vector, artificial chromosomal DNA, or the like. A vector suitable for the host cell to be used can be appropriately selected and used.

Next, the fusion protein according to the present invention can be expressed by introducing the constructed recombinant vector into a host to obtain a transformant and culturing the transformant. The "transformant" referred to in the present invention means a host into which a foreign gene has been introduced. Examples thereof include a host into which a foreign gene has been introduced by introducing plasmid DNA or the like (transformation) and a host into which a foreign gene has been introduced via infection with various viruses and phages (transduction).

The host is not limited and can be appropriately selected as long as it can express the fusion protein according to the present invention after the recombinant vector is introduced. Examples thereof include known hosts such as various animal cells of humans, mice, and the like, various plant cells, bacteria, yeasts, and plant cells.

In a case in which an animal cell is used as a host, for example, human fibroblasts, human fetal kidney cells, HEK293 cells, 293F cells, CHO cells, monkey cells COS-7, Vero cells, mouse L cells, rat GH3 cells, human FL cells, and the like are used. Insect cells such as Sf9 cells and Sf21 cells can also be used.

In a case in which a bacterium is used as a host, for example, *Escherichia coli, Bacillus subtilis,* and the like are used.

In a case in which a yeast is used as a host, *Saccharomyces cerevisiae, Schizosaccharomyces pombe,* and the like are used.

In a case in which a plant cell is used as a host, for example, tobacco BY-2 cells and the like are used.

The method for obtaining the transformant is not limited, and can be appropriately selected in consideration of the combination of types of the host and the expression vector. Preferred examples thereof include the electric perforation method, the lipofection method, the heat shock method, the PEG method, the calcium phosphate method, the DEAE dextran method, and methods of infection with various viruses such as DNA virus and RNA virus.

**In** the obtained transformant, the codon type of the polynucleotide contained in the recombinant vector may be the same as or different from the codon type of the host used, and is not limited.

Further, in the present invention, the method for producing a fusion protein (53X tribody) comprising culturing the above-described transformant and collecting (and purifying) the fusion protein (53X tribody) according to the present invention from the obtained culture can also be provided. Furthermore, the fusion protein (53X tribody) obtained by the production method is also included in the present invention. The methods and conditions for culturing, collection, purification, and the like can be appropriately selected and adopted based on the common general technical knowledge and known findings of those skilled in the art.

### 4. Pharmaceutical Composition

The fusion protein (53X tribody) according to the present invention is useful as an active ingredient contained in a pharmaceutical composition.

Since the fusion protein according to the present invention can have anti-tumor activity as described above, the fusion protein according to the present invention is preferably used for the treatment, prevention, and/or diagnosis of tumors. Therefore, the pharmaceutical composition is useful as a pharmaceutical composition for treating and/or preventing tumors, as well as for diagnosis. In other words, the fusion protein according to the present invention is useful as an active ingredient contained in a tumor therapeutic agent and a tumor diagnostic agent. In the present invention, the treatment of the tumor also includes the meaning of inhibition and suppression of tumor growth, and specifically, for example, in the case of a tumor therapeutic agent, the form of a tumor growth inhibitor and a tumor growth suppressor is also included.

The pharmaceutical composition of the present invention is preferably provided in the form of a pharmaceutical composition containing the fusion protein according to the present invention as an active ingredient and further containing a pharmaceutically acceptable carrier.

The above-described exemplary description of tumor or cancer can be similarly applied to the tumor to which the pharmaceutical composition of the present invention is applied. The tumor to which the pharmaceutical composition of the present invention is applied may be a relapsed cancer or a metastatic cancer, and the pharmaceutical composition (and also the fusion protein according to the present invention) can also be used effectively as a therapeutic agent, a preventive agent, and a diagnostic agent for the relapsed cancer or the metastatic cancer.

Examples of the "pharmaceutically acceptable carrier" include excipients, diluents, bulking agents, disintegrants, stabilizers, preservatives, buffers, emulsifiers, fragrances, colorants, sweeteners, thickeners, flavors. Solubilizers, and other additives. By using one or more of such carriers, pharmaceutical compositions in the form of injections, liquids, capsules, suspensions, emulsionss, syrups, or the like can be prepared. These pharmaceutical compositions can be administered orally or parenterally. Other forms for parenteral administration include injections containing one or more active substances and prescribed by conventional methods. Injections can be produced by dissolving or suspending in a pharmaceutically acceptable carrier such as physiological saline or commercially available distilled water for injection.

The dose of the pharmaceutical composition of the present invention varies depending on the age, sex, body weight, and symptoms of the patient, therapeutic effects, administration method, treating time, type of the fusion protein according to the present invention contained in the pharmaceutical composition, and the like. In general, it can be administered in a range of 600 µg to 6000 mg per adult at a time, but is not limited to this range.

For example, when administered in the form of an injection, a dose of 0.1 µg to 100 mg/kg body weight may be administered to a human patient once to several times per day on average, and preferably can be administered once per 3 days, one week, ten days, or two weeks, or the injection can be administered as a single dose (total number of administrations is once). Examples of the form of administration include intravenous injection, subcutaneous injection, intradermal injection, intramuscular injection, and intraperitoneal injection, but intravenous injection is preferable. **In** addition, the injection may be prepared as a non-aqueous diluent (e.g., vegetable oil such as polyethylene glycol or olive oil, an alcohol such as ethanol, or the like), a suspending agent, or an emulsion. Sterilization of such an injection can be performed by filtration sterilization with a filter, blending of a bactericidal agent, or the like. The injection can be produced in the form of an injection prepared immediately before use. Specifically, the injection can be prepared as a sterile solid composition by a freeze-drying method or the like, and dissolved in sterile distilled water for injection or another vehicle before use.

The present invention also provides the use of the fusion protein according to the present invention for producing a medicament (drug) for treating, preventing, and/or diagnosing a tumor.

The present invention also provides the fusion protein according to the present invention for treating, preventing, and/or diagnosing a tumor.

Furthermore, the present invention provides a method for treating, preventing, and/or diagnosing a tumor, which comprises using (i.e., administering to a subject (patient)) the fusion protein according to the present invention. It also provides the use of the fusion protein according to the invention for treating, preventing, and/or diagnosing a tumor.

### 5. Kit for Diagnosing or Detectng Tumor

The fusion protein according to the present invention can be provided in the form of a kit for treating and/ or preventing a tumor, as well as akit for diagnosing or detecting a tumor. The above description can be similarly applied to specific examples of tumors to be diagnosed or detected.

The diagnosis and detection can be performed, for example, by reacting the fusion protein according to the present invention with a sample collected from a living body (hereinafter, a biological sample) and detecting a signal of the reacted antibody or the like.

The detected signal of the antibody or the like serves as an index of the amount of antigen in the biological sample. In the diagnosis and detection of a tumor using the fusion protein according to the present invention, first, a biological sample collected from a subject as a sample, for example, a tissue piece, blood, or the like to be tested, and the fusion protein (trispecific antibody) according to the present invention are bound by an antigen-antibody reaction. Then, based on the measurement result of the amount of antibody bound, the target antigen amount in the biological sample is measured. The measurement may be carried out according to a known immunological measurement method, and for example, an immunoprecipitation method, an immunoagglutination method, a labeled immunoassay method, an immunoturbidimetric method, a Western blotting method, a flow cytometry method, or the like can be used. In the labeled immunoassay method, the antibody signal is represented by the labeled amount directly detected using the labeled antibody, or an antibody having a known concentration, or a known antibody titer may be relatively represented as a standard solution. In other words, the standard solution and the sample can be measured with a measuring meter, and the signal of the antibody or the like in the biological sample can be relatively expressed with reference to the value of the standard solution. Examples of the labeled immunoassay method include the ELISA method, the EI method, the RIA method, the fluorescence immunoassay (FIA) method, and the chemical luminescence immunoassay. Among them, the ELISA method is particularly preferable in terms of simplicity and high sensitivity.

The state of the tumor can be evaluated or diagnosed using the detection result obtained as described above as an index. For example, when the detection result exceeds a predetermined reference value, it is determined to be positive, and when it is below a predetermined reference value, it is determined to be negative. **In** the case of the positive result, it is judged that some kind of tumore may have developed, and thus, the state of tumor can be evaluated. Here, the state of a tumor means the presence or absence of the affecting tumor or the degree of progression thereof, and includes the development or non-development of tumor, the degree of progression, malignancy, the presence or absence of recurrence, and the like. The presence or absence of metastasis of tumor also can be be

**In** the above evaluation, one state of tumor may be selected, or a plurality of states of tumor may be selected in combination. The presence or absence of tumor can be evaluated based on the obtained detection result by determining whether or not the patient has a tumor with a predetermined reference value as a boundary. Tumor malignancy is an indicator of how advanced the tumor's symptoms are. Based on the detection result, it is also possible to classify and evaluate the stage, or to classify and evaluate early cancer and advanced cancer. For example, it is also possible to evaluate a tumor as early cancer or advanced cancer using the detection result as an index. Tumor metastasis can be evaluated by using the detection result as an index and determining whether or not a neoplasm appears at a site distant from the location of the primary lesion. Tumor recurrence can be assessed by whether the detection result again exceeds a predetermined reference value after an intermittent period or remission.

Further, the diagnosis and detection include, for example, reacting the fusion protein according to the present invention with a sample collected from a living body (e.g., tumor cells and PBMCs derived from a patient), and measuring the signal of the reacted antibody and the like and the cell-killing activity, thereby using the measurement result as an index for determining the efficacy of an antibody and a pharmaceutical product containing the antibody or deciding whether or not to administer the pharmaceutical produc

In addition to including the fusion protein according to the present invention, the kit of the present invention may include a labeling substance, a different antibody, or an immobilization reagent on which the labeling substance is immobilized. The antibody labeled with a substance means an antibody labeled with an enzyme, a radioisotope, a fluorescent compound, a chemiluminescent compound, or the like. In addition to the above-described components, the kit of the present invention may include other reagents for carrying out the detection of the present invention, for example, when the labeling substance is an enzyme-labeling substance, an enzyme substrate (e.g., a color-developing substrate), an enzyme substrate lysate, an enzyme reaction terminator, a diluent for a sample, or the like. Further, various buffers, sterile water, various cell culture vessels, various reaction vessels (e.g., an Eppendorf tube), blocking agents (serum components such as bovine serum albumin (BSA), skim milk, and goat serum), washing agents, surfactants, various plates, preservatives such as sodium azide, and experimental operation manuals (instructions) may be included.

Hereinafter, the present invention will be described in more detail with reference to examples, but the present invention is not limited thereto.

In this Example, in explaining various experiments and results regarding the fusion protein (trispecific tribody) according to the present invention, first, the section of "Results" will be described, and then the section of "Materials and Methods" will be described.

### Example 1

### 1. Results

### Construction and purification of novel 53X tribodies

Novel trispecific multifunctional tribodies were constructed as shown in Figures 1A and 1B and also listed in Table 1 below.

**[Table 1]**

| Name of product | Structure | | Fab | scFV |
|---|---|---|---|---|
| Tb535H | (Fab)5T4 - (scFV) CD3 - (scFV) 5T4 | H-chain derivative | hu5T4 | huOKT3 |
| | | L-chain derivative | hu5T4 | hu5T4 |
| 53L1 | (Fab)5T4 - (scFV) CD3 - (scFV) PD-L1 | H-chain derivative | hu5T4 | huOKT3 |
| | | L-chain derivative | hu5T4 | PD-L1_1 |
| 53L10 | (Fab)5T4 - (scFV) CD3 - (scFV) PD-L1 | H-chain derivative | hu5T4 | huOKT3 |
| | | L-chain derivative | hu5T4 | 10.12 |
| 53D | (Fab)5T4 - (scFV) CD3 - (scFV) PD-1 | H-chain derivative | hu5T4 | huOKT3 |
| | | L-chain derivative | hu5T4 | PD-1_1 |
| 53G | (Fab)5T4 - (scFV) CD3 - (scFV) LAG3 | H-chain derivative | hu5T4 | huOKT3 |
| | | L-chain derivative | hu5T4 | LAG3_1 |
| 53P | (Fab)5T4 - (scFV) CD3 - (scFV) Palibizumab | H-chain derivative | hu5T4 | huOKT3 |
| | | L-chain derivative | hu5T4 | Palibizumab |

In this Example, novel trispecific multifunctional tribodies were generated. The tribody format is a multi-specific antibody including a Fab domain as a scaffold, in which Fd fragment of H-chain (V_{H}+CH₁) and L-chain (V_{L}+C_{L}) can naturally heterodimerize *in vivo,* and additional functions such as scFvs that can be incorporated onto the scaffold. Tb535H is a bi-specific tribody, made up of bivalent 5T4 binding via a Fab domain and a scFv domain and another monovalent CD3 binding via scFv (Patent Literature 1: WO 2016/097408 (A1)). The novel 53X tribodies generated in this Example have binding specificity for three different molecules in a single molecule, 5T4, CD3, and PD-L1 (or PD-1 or LAG3). Specifically, the 5T4-bound scFv arm of an L chain-scFV derivative of Tb535H was replaced with:
a Pd-1_1-derived binding moiety (SEQ ID NO: 22),
a PD-L1_1-derived binding moiety (SEQ ID NO: 45),
a 10.12-derived binding moiety (SEQ ID NO: 56, 75, 76, or 77; where SEQ ID NO: 75, 76, or 77 is an example of a sequence intended to modify any amino acid in SEQ ID NO: 56 for structural stabilization and introduce a cross-link by a disulfide bond in scFv), or an LAG3_1-derived binding moiety (SEQ ID NO: 35); or
a palivizumab-deribed binding moiety (SEQ ID NO: 66) used as a negative control.

As shown in Table 1 above, the constructed novel 53X tribodies according to the present invention are referred to respectively as
53D[(Fab)5T4×(scFV)CD3×(scFV)PD-1(PD-1_1)],
53L1[(Fab)5T4×(scFV)CD3×(scFV)PD-L1(PD-L1_1)],
53L10[(Fab)5T4×(scFV)CD3×(scFV)PD-L1(10.12)],
53G[(Fab)5T4×(scFV)CD3×^{x}(scFV)LAG3(LAG3 _1)], and
53P[(Fab)5T4×(scFV)CD3×(scFV)palivizumab].

The 53P tribody was constructed for use as a negative control over the other 53X tribodies.

### Binding affinity of 53X tribodies for recombinant 5T4 by enzyme-linked immuno sorbent assay (ELISA)

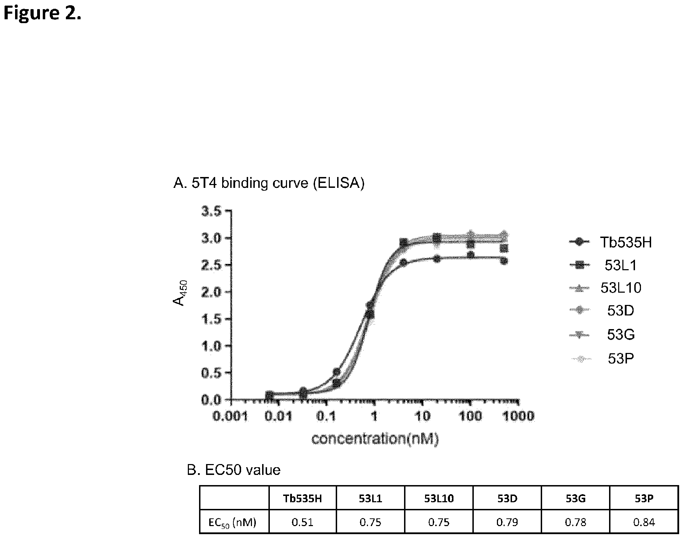

To verify whether the newly created 53X tribodies 53D, 53L1, 53L10, 53G and 53P, each retain the ability to bind to the 5T4 antigen of Tb535H, binding affinity of purified tribodies to the 5T4 protein was examined by ELISA (Figure 2). A recombinant human 5T4 protein (AMSBio) was immobilized onto 96-well ELISA plates. The binding assay of Tb535H and purified novel tribodies was performed by testing at increasing concentrations (0 - 500 nM) were carried out according to the procedure described in Materials and Methods. As shown in Figure 2A, all constructed tribodies showed concentration-dependent binding to 5T4 and Kd (nM) values equal to or greater than Tb535H (Figure 2B). The Tb535H has two 5T4 binding arms derived from Fab and scFv, and the novel 53X tribody constructed has a monovalent 5T4 binding arm derived from a single Fab. Nevertheless, the results show that a single arm (Fab) is sufficient to retain the binding of TB535H to 5T4 in all new 53X tribodies in the same manner.

### Binding affinity of 53X tribodies for recombinant CD3 by ELISA

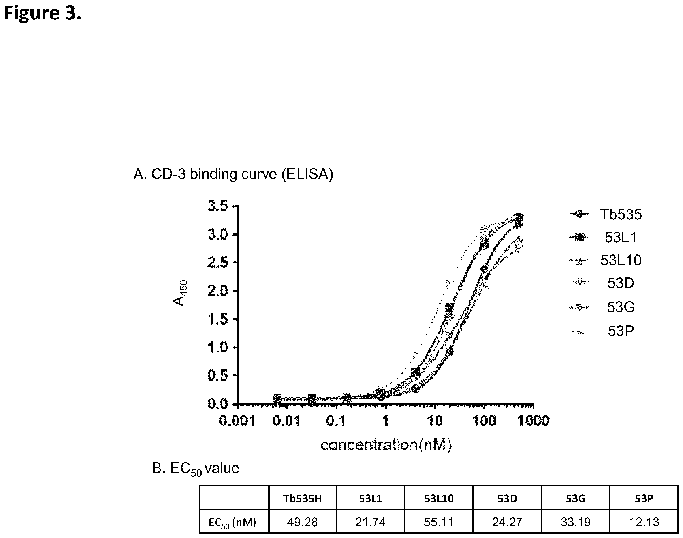

To verify whether the constructed novel 53X tribodies 53D, 53L1, 53L10, 53G and 53P, retain the ability of the Tb535H to bind to CD3 antigen, binding affinity of purified tribodies to recombinant CD3 protein was examined by ELISA (Figure 3). Recombinant human CD3δ/ε heterodimeric protein (AMSBio) was immobilized onto 96-well ELISA plates. The binding assays of Tb535H and purified novel tribodies tested at increasing concentrations (0 - 500 nM) were carried out according to the procedure described in Materials and Methods. As shown in Figure 3A, all constructed tribodies showed concentration dependent CD3 binding with similar Kd (nM) values (Figure 3B). The results suggest the novel tridodies retain the ability of Tb535H to bind to human CD3 protein.

### Binding to recombinant PD-L1 or PD-1 proteins of novel 53X tribodies including a PD-L1 or PD-1 binding arm by ELISA

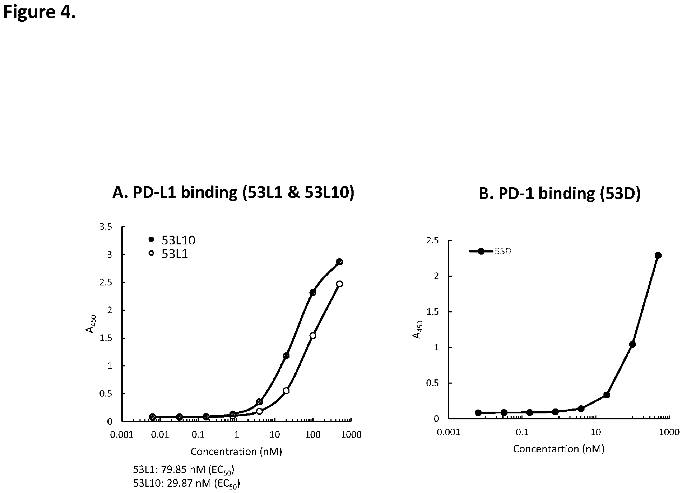

To verify whether the novel tribodies including a binding arm to PD-L1 or PD-1 have a satisfactory binding affinity for their target recombinant proteins, we perfomed ELISA assays on immobilized PD-1 or PD-L1 proteins (Figure 4). Tribodies 53L1 and 53L10 which have a binding arm to PD-L1 showed binding abilities to recombinant PD-L1 protein in a concentration dependent manner with Kd values of 79.85 nM and 29.87 nM, respectively (Figure 4A). The tribody 53D which has a binding arm to PD-1 also showed the binding ability to recombinant PD-L1 protein in a concentration dependent manner (Figure 4B).

### T cell activation bioassays in the presence of 5T4-expressing target cells.

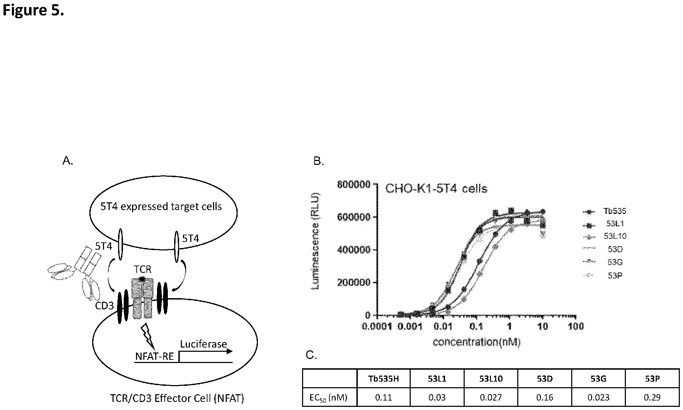

Activation of T cell receptor (TCR)/CD3 on effector cells by novel tribodies and Tb535H was examined on 5T4-expressing CHO-K1 (CHO-5T4) cells used as target cells. The assays were performed by using T cell activation bioassays (NFAT) (Promega, Cat#. J1621). Figure 5A shows the schematic representation of the assay. When cell surface 5T4 on target cells and CD3 on the effector cells are cross-linked with a bi-specific T cell engager, TCR/CD3 transduces intracellular signals resulting in NFAT-RE-mediated luminescence. Genetically engineered Jurkat T cell line which expresses a luciferase reporter driven by a NFAT-response element (NFAT-RE) ("TCR/CD3 effector cells") were co-cultured with CHO-5T4 cells ("target cells") in the presence of increasing concentrations of Tb535H and novel 53X tribodies, as indicated (Figure 5B). Tb535H induced luciferase activity in a concentration dependent manner and EC₅₀ was 0.11 nM. The novel tribodies also induced a concentration-dependent luciferase activity with EC₅₀ values in the range of 0.03 nM - 0.29 nM (Figure 5C). The 5T4-mediated CD3 activation by novel tribodies (with the exception of 53D) was slightly better than that of the Tb535H, however, the EC₅₀ values of novel tribodies fell within almost three-fold (3-fold) lower than that of Tb535H.

### Antagonist effects of 53X tribodies tested by competitive ELISA assay

To verify whether the novel tribodies 53 D, 53L1 and 53L10, derived from PD-1.1 and PD-L1.1 binding domains, respectively, retain the ability of the parental mAbs to interfere in PD-1/PD-L1 interaction, we performed competitive ELISA assays by measuring the binding of biotinylated PD-L1 ligand to immobilized PD-1 receptor in the absence or in the presence of a molar excess (5:1 M/M) of the tribodies 53 D, 53L1, 53L10 or their parental mAbs PD-L1_1, 10.12, and PD-1_1 as positive controls in parallel assays. Human IgG4 isotype was used as a control. As shown in Figure 6 A and B, the binding of biotinylated PD-L1 ligand was significantly reduced in the presence of the tribodies with respect to the binding signal of the biotinylated PD-L1 used alone, thus suggesting that the novel tribodies retain the ability of the parental mAbs to interfere in the interactions of PD-L1 with PD-1.

Similarly, the ability of tribody 53G to interfere with LAG-3/MHCII (HLA-DRA) interaction was analyzed from competitive ELISA assay. To this aim, LAG-3-His-GST recombinant protein was coated on plates at the concentration of 50 nM, the tribody 53 G or its parental mAb LAG-3_1 were added at a saturating concentration of 2 µM and the binding between the biotinylated-MHCII protein (700 nM) and the LAG3-His-GST recombinant protein was measured. As shown in Figure 6 C, the binding beetween biotinylated MHCII and the LAG3-His-GST recombinant protein was strongly reduced in the presence of the tribody 53G or the parental LAG-3_1 (mAb), used as control, with respect to the binding signal in the case of treatment with the biotinylated MHC II alone.

### Cytotoxic activity of novel tribody in co-culture of human cancer cells and human lymphocytes in vitro

To examine whether novel tribodies targeting PD-L1, PD-1, and LAG-3 effectively induce lymphocyte activation for cancer cells in human cancer cells and lymphocytes, their effects were investigated in a co-culture system. To this end, MDA-MB-231 breast cancer cells that highly express PD-L1 and 5T4 and A-549, a human alveolar basal epithelial adenocarcinoma cell line, were co-cultured with human peripheral blood mononuclear cells (hPBMCs) and incubated at 37°C for 48 hours in the absence or presence of 53D, 53G, 53L1, and 53L10 (effector:target ratio = 5:1). In parallel, the tribodies (53P) derived from the same concentrations of parental tribodies TB535H and TB535H but lacking immunomodulatory antibody fragments, the above parental mAbs PD-L1.1, 10.12, PD-1.1, and LAG-3.1, and the effect of combined use of Tb535H and parental mAb were also examined After incubation, cytolysis was measured by LDH released into the cell supernatant using lactate dehydrogenase (LDH) assay. The results showed that all novel tribodies, except 53P used as a negative control, induced tumor cytolysis with greater efficacy than in combination with the parental mAb or TB535H (see Figures 7 and 8). Specifically, all tribodies at least doubled LDH release levels in combination with the corresponding parental mAbs and TB535H tribodies, and in the cases of 53L1 and 53L10, about 80% to 90% cytolysis was achieved at a concentration of 1 nM.

To clarify whether the enhancement of tumor cytolysis by the novel tribody correlates with increased activation of lymphocytes, the concentration of IFN-γ (functional marker for hPBMC activation) released in the culture supernatants of those co-cultures was measured, and the effect of the novel tribody was compared with the effect of TB535H in combination with the parental mAb. As shown in Figures 9, IFN-γ levels were much higher in the supernatants of the co-cultures of MDA-MB-231 and hPBMCs treated with the novel tribodies (TRBs) with respect to those observed in the treatments with their parental tribody TB535H, their parental mAbs PD-L1.1, 10.12, PD-1.1, and LAG-3.1, or their combinations.

Thus, these results clearly confirm the validity of the strategy to insert an immunomodulatory moiety in the parental TB535H to increase its anti-tumor efficacy.

### In vivo anti-tumor efficacy of novel 53X tribodies

The *in vivo* anti-tumor efficacy of the novel tribodies was evaluated in an A549 (human alveolar basal epithelial adenocarcinoma cell line) xenograft mouse model. Expression of 5T4 and PD-L1 on the cell surface in A549 cells was confirmed by flow cytometric analysis (data not shown). Human PBMCs were activated by Dynabeads Human T-activator CD3/CD28 for 4 days prior to transplantation (activated PBMCs). A549 lung cancer cells were subcutaneously transplanted into immunodeficient NOD-SCID mice with the same number of activated human PBMCs (Day 0). Intravenous administration of the tribodies shown in the figure was performed every other day from Day 0 to Day 10 (total of 6 administrations) (Figure 10). In the Tb535H treatment group, significant tumor growth inhibition was observed at a dose of 20 µg/mouse compared to vehicle treatment (p < 0.05), but no significant tumor growth inhibition was observed at a dose of 2 µg/mouse (Figure 10A). Meanwhile, the anti-tumor activity of 53L1 (20 µg/mouse) was clearly stronger than that of Tb535H used at the same dose (Figure 10B). Furthermore, in the 53L10 treatment group, significant tumor growth inhibition of A549 tumor was observed by treatment at both 2 µg/mouse and 20 µg/mouse doses compared to vehicle treatment (Figure 10C). Tumor growth inhibition (TGI (%)) in the 53L10 (2 µg/mouse) treatment group on Day 50 reached 41.5% compared to the vehicle treatment group (p < 0.05), and 100% TGI was observed (p < 0.05) in the 20 µg/mouse treatment group. Complete tumor regression was observed in all mice on the final day of the study (Day 50) in the 53L10 (20 µg/mouse) treatment group.

Tumor regression by treatment with 53L10 was reproduced also in a different set of the study (Figure 11). The growth of A549 subcutaneous tumors was significantly inhibited by 53L10 treatment in a dose dependent manner and the complete tumor regression in all mice even at the final day (day 42) was observed in 53L10 (20 µg/mouse) treatment group.

Anti-tumor activities of 53D and 53G, respectively, were also evaluated in the same models in comparison with the control 53P tribody (Figure 12). At 53D, tumor growth inhibition was enhanced in the 20 µg/mouse treatment group compared with 53P (Figure 12). Tumor growth inhibition by 53G treatment was enhanced at both 2 µg/mouse and 20 µg/mouse doses compared to 53P treatment. Significant tumor regression was observed during the study period in the 53G (20 µg/mouse) treatment group.

### 2. Materials and Methods

Cell cultures MDA-MB-231 breast cancer cells were cultured in Dulbecco's Modified Eagle's Medium (DMEM, Gibco, Life Technologies). A-549 lung cancer cells were cultured in Kaign's Modification of Ham's F-12 Medium (F-12K, American Type Culture Collection). Cell lines were purchased from the American Type Culture Collection (ATCC) and cultured in a humidified atmosphere containing 5% CO2 at 37°C. The media were supplemented with 10% (vol/vol) inactivated fetal bovine serum (FBS, Sigma) and were used after addition of 50 U/mL penicillin, 50 µg/mL streptomycin, 2 mM L-glutamine (all from Gibco, Life Technologies).

### Antibodies and human recombinant proteins

The anti-PD-L1 antiboies (PD-L1_1 and 10.12), anti-PD-1 antibody (PD-1_1), anti-LAG-3 antibody (LAG3_1), and human IgG4 isotype control antibody were purified as previously described (Patent Literature 2and Non Patent Literature 10 above). Tb535H tribody was prepared as described in Patent Literature 1 above. Recombinant human PD-L1/Fc, PD-1/Fc and human LAG3/Fc proteins were purchased from R & D Systems. Human LAG-3/His-GST and Human HLA class II histocompatibitily antigen-DRA was purchased from Cusabio. Human 5T4/His was purchased from ACROBiosystems and Human CD3δ/ε heterodimer protein was purchased from AMSBIO.

### Production and purification of novel 53X tribodies

Expression plasmid encoding H-chain derivatives in combination with the expression plasmid encoding L-chain derivatives for each tribody (53D, 53G, 53L1, 53L10, and 53P) were transiently transfected and expressed, thereby producing a heterodimer in Expi293 cells. After 5 days post-transfection, each tribody was purified from cell culture supernatants by His-tag affinity chromatography followed by gel filtration chromatography. This process recovers the tribody heterodimer while limiting impurities such as a product variant. The purity of the tribodies were analyzed by SDS-PAGE, and size-exclusion chromatography (SEC) analysis was performed to evaluate the tribody heterodimer purity and freeze-thaw stability. It was confirmed > 95% L/Fd chain tribody heterodimer, and low levels of endotoxins by a LAL based method. Tribodies were sterilized by 0.2 µm filtration, aliquoted and stored at -80°C.

### Binding assay (ELISA)

To confirm the binding specificity of the novel generated tribodies, ELISA assays were performed on human chimeric recombinant target proteins (5T4/His, CD3δ/ε, PD-L1/Fc, PD-1/Fc, or LAG-3/Fc coated at 1-5 µg/mL on 96-well plates).

The ELISA assays on coated recombinant target protein were performed by blocking the coated plates with 5% skim milk in PBS for 1 hour at 37°C, the purified tribodies were added at different concentrations to the plates in 3% BSA (bovine serum albumin) in PBS and incubated for about 90 minutes hours at room temperature by gently shaking. After extensive washes with PBS, the plates were incubated with HRP-conjugated anti-human Ig κ antibody (Southern Biotech) or with anti-His-HRP-conjugated antibody (MBL) for 1 hour, washed again and incubated with TMB reagent (Sigma-Aldrich) for 10 min before terminating the reaction with an equal volume of 1 N hydrochloric acid (HCl).

Absorbance at 450 nm was measured by an Envision plate reader (PerkinElmer Co., Ltd., 2102 Waltham, MA, USA).

### Competitive ELISA assay

In order to investigate the ability of the novel generated tribodies, in comparison with the corresponding parental mAbs, to compete in the PD-L1/PD-1 or LAG-3/MHC II (HLA-DRA) binding, competitive ELISA assays were performed by testing the binding of each biotinylated chimeric protein (PD-L1/Fc or MHCII) to PD-1 or LAG-3/GST-His in the absence or in the presence of the unlabelled competitive tribodies. To this aim, NuncTM flat-bottom 96-well plate were coated with PD-1 or LAG-3 proteins. Then, the coated plates were pre-incubated with the unlabelled anti-PD-L1 antibodies, anti-PD-1 antibodies or anti-LAG-3 antibodies at saturating concentrations for 2 hours at room temperature (5:1 for anti-PD-L1 antibody or anti-PD-1 antibody and 3:1 for anti-LAG-3), and then further treated with the biotinylated PD-L1 or MHCII chimeric proteins, which were added to the plate at the same concentrations of the competitive antibodies for 2 hours at room temperature. For the detection of bound biotinylated proteins, HRP-conjugated Streptavidin (Biorad) was added to the plate for 30 minutes and then the absorbance measured as mentioned above.

### Cell viability and cytotoxicity assay (LDH Detection)

Human Peripheral Blood Mononuclear Cells (hPBMCs) were isolated from blood of healthy donors by using Greiner Leucosep^{®} tube (Sigma-Aldrich) and cryopreserved by following the manufacturer's instructions. Cryopreserved cell vials were thawed out, collected for resting and counted before use, as previously reported (Non Patent Literature 11 above).

To measure the cytotoxic activity of the novel generated tribodies, target tumor cells were co-cultured with hPBMCs (effector: target ratio 5:1) and treated with the tribodies used at increasing concentrations, or with the parental mAbs and their corresponding combinations used in parallel assays at the same concentrations. To this aim, tumor cells were plated in 96-well flat-bottom plates at the density of 1 × 10⁴ cells/well, for 16 hours. Then, hPBMCs isolated from healthy donors were added in the absence or in the presence of the tribodies or antibodies and incubated at 37 °C for 48 hours. Untreated cells or cells treated with a human IgG4 isotype control were used as negative controls.

The lysis of the target cells was measured by detecting the levels of the lactate dehydrogenase (LDH) released in the supernatant of the co-cultures, by using the LDH detection kit (Thermofisher Scientific), following the manufacturer's recommendations. Cell lysis was analyzed by measuring the increase rate of LDH in the presence of each treatment, with respect to the amount present in the supernatant of co-cultures untreated or treated with the human IgG4 isotype control antibodies and expressed as percentage.

### Cytokine secretion assay

The secretion of human IL-2 and IFNγ in supernatants of co-cultures of tumor cells with hPBMCs, or in the supernatants of hPBMCs treated with the tribodies and their corresponding parental mAbs, was evaluated by DuoSet ELISA kit assays. Briefly, after treatments culture supernatants were centrifuged and the cytokines quantified by using the IL-2 and IFNγ kits (from R&D Systems), according to the producer's recommendations. Concentration values were converted in pg/mL and calculated as the mean of at least three determinations.

### T cell activation bioassay

TCR/CD3 activation in the presence of 5T4-expressing cells was performed by T cell activation bioassay (NFAT) (Promega, Cat#. J1621) in accordance with manufacture's instruction. Briefly, genetically engineered Jurkat T cell line which expresses a luciferase reporter driven by an NFAT-response element (included in the kit) were co-cultured with 5T4 expressing CHO-K1 cells in the presence of various concentration of Tb535H and novel 53X tribodies. After 4 hr incubation at 37°C, Bio-GloTM reagent (included in the kit) was added, and luminescence was measured by luminometer.

### In vivo efficacy test

All animal experiments in this Example were approved and conducted in accordance with the Institutional Animal Care and Use Committee guidelines of Chiome Bioscience Inc. Five to 6 weeks female NOD/ShiJic-scidJcl mice (NOD-SCID) (Japan CLEA) was used for efficacy study. A549 human alveolar lung adenocarcinoma cell lines were harvested from cell culture dishes by trypsinization and washed with PBS and prepared cell suspension. Human PBMCs (Cellular Technology Limited) were cultured *in vitro* in the presence of Dynabeads human T-activator (CD3/CD28) (Veritas) for 4 days before inoculation of A549 cells into mice. Cell suspensions of 5 × 10⁶ A549 cells were mixed with equal number of activated hPBMCs (the ratio of implanted activated hPBMCs to A549 cancer cells was 1:1). Mixed cell suspensions were subcutaneously transplanted in right flank of NOD-SCID mice (day 0) and Intravenous treatment of indicated test article at the indicated dosage were performed at day 0, 2, 4, 6, 8, 10 (total 6 treatment) after the cell transplantation. Tumor growth was measured with calipers in 2 perpendicular dimensions of tumor, and tumor volume (mm³) was calculated using the calculation formula (width² x length) x 3.14/6. Tumor volume was expressed as mean ± standard deviation (SD). The Dunnett's test (vs vehicle treatment group) was used for statistical significance difference: *p < 0.05.

### Example 2

### 1. Results

### Comparison of in vivo anti-tumor efficacy of the novel tribody 53L10 and Tb535H alone and the administration of Tb535H in combination with pembrolizumab

Further, the *in vivo* anti-tumor efficacy in the case of administering 53L10 alone, Tb535H alone, and Tb535H in combination with pembrolizumab (anti-human PD-1 antibody, MSD K.K.) was compared and investigated in an A549 (human alveolar basal epithelial adenocarcinoma cell line) xenograft mouse model (Figure 13). A cell suspension containing 5 × 10⁶ A549 cells mixed with the same number of activated human PBMCs was subcutaneously transplanted into the right flank of NOD-scid mice (Day 0). From the day of transplantation, administration of vehicle (PBS), Tb535H (6, 12, and 20 µg/mouse), or 53L10 (6, 12, and 20 µg /mouse) into the tail vein was performed every other day for a total of 6 times (Days 0, 2, 4, 6, 8, and 10). Administration of pembrolizumab (200 µg/mouse) into the tail vein was performed 3 times in total on Day 0, Day 4, and Day 8. For administration of Tb535H in combination with pembrolizumab, Tb535H was combined with pembrolizumab at each dose. Tumor volume was measured 3 days after transplantation and thereafter twice a week until 50 days after transplantation.

Figure 13A shows the results of comparing the *in vivo* anti-tumor efficacy of Tb535H and 53L10, and Figure 13B shows the results of comparing the *in vivo* anti-tumor efficacy of 53L10 and the administration of Tb535H in combination with pembrolizumab. In the Tb535H treatment group, significant tumor growth inhibition was observed in the 20 µg/mouse treatment group compared with the vehicle (PBS) treatment group. The TGI on the final day of the test (Day 50) was 36.5% (*P < 0.05) (Figure 13A).

Meanwhile, in the 53L10 treatment group, a tumor growth inhibition effect was observed depending on the dose, and significant tumor growth inhibition was observed in all treatment groups compared with the vehicle (PBS) treatment group (*P < 0.05). In addition, a significant decrease in tumor volume was observed in all 53L10 treatment groups compared with the Tb535H treatment groups (6 µg/mouse, 12 µg/mouse, and 20 µg/mouse, respectively). Tumor growth inhibition (TGI (%)) on the final day of the test (50 days) for the 53L10 treatment group was 64.8% for the 6 µg/mouse treatment group, 76.0% for the 12 µg/mouse treatment group, and 95.7% for the 20 µg/mouse treatment group. In the 53L10 (20 µg/mouse) treatment group, complete tumor regression was observed in 5 out of 8 individuals on the final day of the test (Day 50) (Figure 13A).

Next, a comparison was made between the group treated with Tb535H in combination with pembrolizumab and the group treated with 53L10 tribody alone (Figure 13 B). No significant anti-tumor efficacy was observed in the pembrolizumab (200 µg/mouse) treatment group compared with the vehicle (PBS) treatment group (Figure 13B). In the group treated with Tb535H in combination with pembrolizumab, no significant anti-tumor efficacy was observed in both the Tb535H (6 µg/mouse) + pembrolizumab (200 µg/mouse) treatment group and the Tb535H (12 µg/mouse) + pembrolizumab (200 µg/mouse) treatment group compared with the vehicle (PBS) treatment group (Figure 13B). In the Tb535H (20 µg/mouse) + pembrolizumab (200 µg/mouse) treatment group, significant tumor growth inhibition was observed compared with the vehicle (PBS) treatment group. The TGI on the final day of the study (Day 50) was 38.0% ( *P < 0.05) (Figure 13B).

Meanwhile, as mentioned above, in the 53L10 treatment group, a tumor growth inhibition effect was observed depending on the dose, and significant tumor growth inhibition was observed in all treatment groups compared with the vehicle (PBS) treatment group (*P < 0.05). In addition, a significant decrease in tumor volume was observed in all 53L10 treatment groups compared with the Tb535H + pembrolizumab treatment group (Tb535H: 6 µg/mouse, 12 µg/mouse, and 20 µg/mouse). The TGI on the final day of the test (50 days) for the 53L10 treatment group was 64.8% for the 6 µg/mouse treatment group, 76.0% for the 12 µg/mouse treatment group, and 95.7% for the 20 µg/mouse treatment group. In the 53L10 (20 µg/mouse) treatment group, complete tumor regression was observed in 5 out of 8 individuals on the final day of the test (Day 50)

(Figure 13B).

### Creation of 53L10-M13

It is known that when producing therapeutic antibodies that can be administered to humans using antibodies, antibody-binding fragments, and the like, a chemical modification often occurs after an antibody is purified during the production process. Dealing with such a chemical modification is recognized in the art as important in ensuring the quality and safety of a purified antibody. Specifically, when there is an amino acid sequence motif that is susceptible to modifications such as N-glycan modification, protein cleavage, deamidation, racemization, and oxidation in the sequence including the CDR region of an antibody, this will lead to a decrease in the quality of the purified antibody. As amino acid sequence motifs that are susceptible to modifications, for example, motifs containing asparagine residues and aspartic acid residues are known (e.g., Sydow et al., (2014). Structure-based prediction of asparagine and aspartate degradation sites in antibody variable regions. PLoS ONE, 9(6)). Showing that it is possible to modify the amino acid motifs of antibodies while preserving their activity will further enhance the usefulness of antibodies.

The 62nd aspartic acid residue (D) of scFv (10.12) (SEQ ID NO: 56) was mutated to a serine residue (S), thereby creating scFv (10.12)-M13 (SEQ ID NO: 108). This is obtained by replacing the heavy chain CDR-H2 (SEQ ID NO: 59) contained in scFv (10.12) (SEQ ID NO: 56) with CDR-H2#2 (SEQ ID NO: 49). The 53L10_L chain derivative (SEQ ID NO: 107) containing scFv (10.12)-M13 (SEQ ID NO: 108) was co-expressed with the H chain derivative of SEQ ID NO: 1 in Expi293 cells by the method described above, and the 53L10 mutant (53L10-M13) was purified from the culture supernatant.

### Binding affinity of 53L10-M13 for recombinant 5T4 by ELISA

The binding affinity of purified 53L10-M13 to 5T4 antigen was examined by ELISA (Figure 14). A recombinant human 5T4 protein (ACRO Biosystems) was immobilized in a 96-well plate using the same method as that performed in Figure 2 (Example 1). According to the described procedure, the binding affinities of 53L10-M13 and 53L10 to human 5T4 were determined at increasing concentrations (0-500 nM). As shown in Figure 14A, 53L10-M13 (○) showed binding to 5T4 in a concentration-dependent manner. The curve showing the binding affinity of 53L10-M13 to 5T4 overlapped with the curve showing the binding affinity of 53L10 (●) to 5T4. The EC₅₀ values were 0.58 nM for 53L10-M13 and 0.53 nM for 53L10 (Figure 14B).

### Binding affinity of 53L10-M13 for recombinant CD3 by ELISA

The binding affinity of purified 53L10-M13 to recombinant CD3 protein was examined by ELISA (Figure 15). A recombinant human CD3δ/ε heterodimer protein (ACRO Biosystems) was immobilized in a 96-well plate using the same method as that performed in Figure 3. According to the procedure described in "Materials and Methods" later, the binding assay of 53L10-M13 and 53L10 to human CD3 protein was performed at increasing concentrations (0-500 nM). As shown in Figure 15A, both 53L10-M13 (○) and 53L10 (●) showed binding activity to CD3 protein in a concentration-dependent manner, with EC₅₀ of 53L10 being 39.9 nM and EC₅₀ of 53L10-M13 being 65.5 nM (Figure 15B).

### Binding affinity of 53L10-M13 for recombinant PD-L1 by ELISA

The binding affinity of purified 53L10-M13 to recombinant PD-L1 protein was examined by ELISA (Figure 16). A recombinant human PD-L1/Fc protein (R&D Biosystems) was immobilized in a 96-well plate using the same method as that performed in Example 4. According to the procedure described in "Materials and Methods" later, the binding assay of 53L10-M13 and 53L10 to human PD-L1 protein was performed at increasing concentrations (0-500 nM). As shown in Figure 16A, 53L10-M13 (○) showed binding to PD-L1 in a concentration-dependent manner. The curve showing the binding affinity of 53L10-M13 to PD-L1 overlapped with the curve showing the binding affinity of 53L10 (●) to PD-L1. The EC₅₀ values were 36.9 nM for 53L10-M13 and 34.4 nM for 53L10 (Figure 16B).

### T cell activation bioassays in the presence of 5T4-expressing target cells of 53L10-M13

Next, a T cell activation bioassay (NFAT) (Promega, Cat. No. J1621) was used for examining the activation of T cell receptor (TCR)/CD3 on effector cells by 53L10-M13 in the presence of 5T4-expressing target cells by the same method as that performed in Figure 5 (Figure 17). 53L10 was also examined at the same time for comparison. When cell surface 5T4 on target cells and CD3 on effector cells are cross-linked with 53L10-M13 or 53L10, TCR/CD3 transduces intracellular signals, resulting in NFAT-RE-mediated luminescence. A genetically engineered Jurkat T cell line ("TCR/CD3 effector cells") expressing a luciferase reporter driven by an NFAT response element (NFAT-RE) was co-cultured with CHO-5T4 cells ("target cells") in the presence of 53L10-M13 and 53L10 at increasing concentrations as shown (Figure 17A). Both 53L10-M13 (○) and 53L10 (●) induce luciferase activity in a concentration-dependent manner, and the curves showing the concentration-dependent increase in luciferase activity overlap (Figure 17A), with EC₅₀ of 53L10-M13 being 0.016 nM and EC₅₀ of 53L10 being 0.010 nM (Figure 17B).

The above results show that the antigen binding activity (5T4, CD3, and PD-L1) of 53L10-M13 and the activation ability of TCR/CD3 complex on effector cells in the presence of 5T4-expressing target cells are equivalent to those of 53L10.

### T cell activation by 53L10-M13 and Tb535H in co-culture of A549 cells (5T4-positive PD-L1-positive human cancer cell line) and human peripheral blood mononuclear cells (hPBMCs)

Whether 53L10-M13 could efficiently activate T cells in the presence of target tumor cells was examined by measuring the expression of T cell activation markers (CD25, CD69) on CD3-positive cells. Tb535H was also examined at the same time for comparison. As the target tumor cells, A549 cells of a 5T4-positive PD-L1-positive human cancer cell line were first tested (Figure 18A). According to the procedure described in "Materials and Methods" later, target cells and hPBMCs (Cellular Technology Limited; lots. HHU20211125 and HHU20220224 and lot. HHU20220329, hereinafter referred to as "PBMC1 and PBMC2" and "PBMC3," respectively) were co-cultured (effector : target ratio = 10:1), following which 24 hours after addition of 0.001 pM to 10 nM (10-fold common ratio) of 53L10-M13 or Tb535H, the expression of CD3 and CD69 was measured, and 48 hours later, the expression of CD3 and CD25 was measured by flow cytometry. The percentage of CD69-positive cells in the CD3-positive cell population (Figure 18B) and the percentage of CD25-positive cells in the CD3-positive cell population (Figure 18C) were calculated. As a result, it was shown that both 53L10-M13 (●, ■, ▲) and Tb535H (○, □, △) induced the expression of CD25 and CD69 on effector cells in a concentration-dependent manner; however, for both hPBMC lots, 53L0-M13 more strongly induced the expression of T cell activation markers at the same concentration than Tb535H (Figures 18B and 18C). It was shown that comparing 53L10-M13 and Tb535H for EC₅₀ that could be calculated, the CD69 expression level was 34.9 to 99.1 pM for 53L10-M13 and 100.2 to 1548.0 pM for Tb535H, which was low for 53L10-M13 in all hPBMC lots. Meanwhile, also regarding the maximum response (Eₘₐₓ), the CD69 expression level was 34.9% to 57.1% for 53L10-M13 and 22.9% to 42.5% for Tb535H, which was higher for 53L10-M13 than for Tb535H in all hPBMC lots. These results showed that T cell activation was enhanced in 53L10-M13 compared with Tb535H.

### T cell activation by 53L10-M13 and Tb535H against hPBMC (in the absence of human cancer cells)

Meanwhile, T cell activation by 53L10-M13 and Tb535H was also examined in the absence of target tumor cells. In the absence of target tumor cells, 0.01 to 1,000 nM (10-fold common ratio) of 53L10-M13 or Tb535H was added to hPBMCs. As a result, it was shown that although the marker expression was slightly higher in 53L10-M13 (●, ■, ▲) than in Tb535H (○, □, △) in a high concentration range of 10 nM or more, in both cases, the expression of T cell activation markers (CD25, CD69) on CD3-positive cells in the absence of target tumor cells remains lower than in the presence of target tumor cells, indicating that T cells are hardly activated.

The above results showed that 53L10-M13 activates T cells in the presence of A549 cells, which are target tumor cells, and also causes higher T cell activation than Tb535H in the presence of A549 cells.

### T cell activation by 53L10-M13 and Tb535H in co-culture of MCF-7 cells (5T4-positive PD-L1-negative human cancer cell line) and hPBMCs

Next, T cell activation by 53L10-M13 and Tb535H in co-culture of MCF-7 cells of a human breast cancer cell line and hPBMCs was investigated using the same method. MCF-7 cells were used as 5T4-positive PD-L1-negative target tumor cells (Figure 20A). As a result, both 53L10-M13 (●, **■** , ▲) and Tb535H (○, □, △) induced the expression of CD25 and CD69 on effector cells in a concentration-dependent manner; however, unlike the case of using A549 cells expressing both 5T4 and PD-L1 as target tumor cells, the curves showing the concentration-dependent increase in the expression of T cell activation markers overlapped for both CD69 (Figure 20B) and CD25 (Figure 20C) in 53L10-M13 and Tb535H. EC₅₀ in CD69 expression was 0.10 to 2.85 pM for 53L10-M13 and 2.63 to 5.49 pM for Tb535H. EC₅₀ in CD25 expression was 0.49 to 6.78 pM for 53L10-M13 and 12.57 to 50.79 pM for Tb535H. In both cases, 53L10-M13 showed slightly lower values. Meanwhile, Eₘₐₓ in CD69 expression was 41.85% to 50.68% for 53L10-M13 and 42.56% to 50.41% for Tb535H. Eₘₐₓ in CD25 expression was 19.99% to 24.21% for 53L10-M13 and 27.30% to 29.49% for Tb535H. Both were at the same level. It was shown that when 5T4-positive PD-L1-negative MCF-7 cells are used as target tumor cells, the T cell activation ability of 53L10-M13 and Tb535H is almost the same (Figures 20B, 20C, and 20D).

These results suggest that enhancement of T cell activation by 53L10-M13 compared with Tb535H is a phenomenon observed under conditions where target tumor cells express PD-L1, and that in tumors that do not express PD-L1, the same level of T cell activation as Tb535H, which has a bivalent binding site for 5T4, is induced. Therefore, the validity of the strategy of 53L10 and 53L10-M13 to enhance tumor immunity by adding an anti-PD-L1 binding arm to Tb535H against target tumor cells that express both 5T4 and PD-L1 is positively supported. In addition, it is shown that 53L10 and 53L10-M13 have the same tumor immunity ability as Tb535H, even against tumor cells that do not express PD-L1.

### Cytokine production with hPBMCs in the presence and absence of A549 cells (5T4-positive PD-L1-positive human cancer cell line) by 53L10-M13 and Tb535H

Subsequently, cytokine production from hPBMCs by 53L10-M13 and Tb535H was measured in the presence and absence of A549 cells (hPBMCs alone). After 48 hours, the culture supernatant obtained in the above T cell activation test was collected. According to the procedure described in "Materials and Methods" later, the concentrations of four cytokines secreted from T cells, human TNFα, human IL-2, human IL-6, and human interferon γ, in the culture supernatant were measured. As a result, in the absence of A549 cells (hPBMCs alone), neither 53L10-M13 nor Tb535H caused concentration-dependent cytokine production. On the other hand, in the presence of A549 cells, concentration-dependent production of IL-6 was observed in Tb535H, but no concentration-dependent production of other cytokines was observed. In 53L10-M13, cytokine production was observed for all four cytokines in a concentration-dependent manner of added 53L10-M13 (Figures 21A-21D (A: human TNFα, B: human IL-2, C: human IL-6, D: Human interferon γ)). Cytokine production by 53L10-M13 was caused by adding 53L10-M13 at 100 pM or more, similar to T cell activation (Figures 18B and 18C). Production of all four cytokines was more enhanced by adding 53L10-M13 than by adding Tb535H. This also showed that 53L10-M13 induces T cell activation more strongly than Tb535H in the presence of A549 cells.

### Cytotoxicity test of 53L10-M13 and Tb535H against A549 cells in co-culture of A549 cells (5T4 positive PD-L1 positive human cancer cell line) and hPBMCs (without activation treatment)

Next, whether 53L10-M13 could efficiently induce cytotoxicity against target tumor cells was verified. A549 cells were co-cultured with hPBMCs under the same conditions as the T cell activation test (effector : target ratio = 10:1), and the cytotoxicity was examined when 53L10-M13 and Tb535H were added at 0.001 pM to 10 nM (10-fold common ratio). According to the procedure described in "Materials and Methods" later, hPBMCs were added to A549 cells whose cell membranes had been previously labeled with PKH67, 53L10-M13 and Tb535H were added at varying concentrations, and the cells were collected after culturing for 48 hours. Dead cells were stained with propidium iodide (PI), and the number of PKH67-positive PI-negative cells was measured using a flow cytometer, thereby determining cytotoxicity (%). As a result, it was shown that both 53L10-M13 (●, ■, ▲) and Tb535H (○, □, △) induced cytotoxicity in a concentration-dependent manner; however, 53L10-M13 induced higher cytotoxicity than Tb535H at the same concentration in all hPBMC lots (Figure 22A). Regarding PBMC3, for which EC₅₀ and Eₘₐₓ could be calculated for both 53L10-M13 and Tb535H, EC₅₀ was 11.5 pM for 53L10-M13 and 33.7 pM for Tb535H, which was low for 53L10-M13, and Eₘₐₓ was 85.2% for 53L10-M13 and 69.4% for Tb535H, which was higher in 53L10-M13, showing that 53L10-M13 enhanced cytotoxicity than Tb535H. In Figure 22B, "n.c." stands for "not calculated."

### Cytotoxicity test of 53L10-M13 and Tb535H against A549 cells in co-culture of A549 cells (5T4 positive PD-L1 positive human cancer cell line) and activated hPBMCs

Furthermore, the induction of cytotoxicity of 53L10-M13 and Tb535H against target cancer cells was also investigated in activated hPBMCs. hPBMCs that were previously activated with anti-CD3 antibody/anti-CD28 antibody solid-phase magnetic beads were co-cultured with A549 cells (effector : target ratio = 1:1), and the cytotoxicity was examined when 53L10-M13 (●, ▲) and Tb535H (○, △) were added. As a result, although both 53L10-M13 and Tb535H induced concentration-dependent cytotoxicity in activated hPBMCs, Tb535H induced only a little more than 50% cytotoxicity at most, while on the other hand, in 53L10-M13, a maximum of 90% or more cytotoxicity was induced (Figure 23A). Regarding PBMC3, for which EC₅₀ and Eₘₐₓ could be calculated for both 53L10-M13 and Tb535H, EC₅₀ was 15.3 pM for 53L10-M13 and 10.4 pM for Tb535H, which was slightly lower for Tb535H. At the same time, Eₘₐₓ was 90.0% for 53L10-M13 and 65.4% for Tb535H, which was significantly higher in 53L10-M13, showing that 53L10-M13 enhanced cytotoxicity than Tb535H also in activated PBMCs (Figure 23B). In Figure 23B, "n.c." stands for "not calculated."

These results showed that 53L10-M13 not only enhanced T cell activation more than Tb535H in the presence of target tumor cells, but also significantly enhanced the cytotoxicity of activated T cells. This positively supports the validity of the strategy of 53L10 and 53L10-M13 to enhance tumor immunity against target tumor cells by adding an anti-PD-L1 binding arm to Tb535H.

### 2. Materials and Methods

### Cell culture

A549 human lung cancer cells were cultured in Kaign's Modification of Ham's F-12 medium (F-12K, FUJIFILM Wako Pure Chemical Corporation). Cell lines were purchased from the American Type Culture Collection (ATCC) and cultured in a humidified atmosphere containing 5% CO₂ at 37°C. The media were supplemented with 10% (vol/vol) inactivated fetal bovine serum (FBS, Sigma) and were used after addition of 50 U/mL penicillin and 50 µg/mL streptomycin. MCF-7 human breast cancer cells were cultured in Minimum Essential Medium Eagle medium (EMEM, Sigma-Aldrich). Cell lines were purchased from the American Type Culture Collection (ATCC) and cultured in a humidified atmosphere containing 5% CO₂ at 37°C. The media were supplemented with 10% (vol/vol) inactivated fetal bovine serum (FBS, Sigma) and were used after addition of 50 U/mL penicillin, 50 µg/mL streptomycin, and 0.01 mg/ml human recombinant insulin (FUJIFILM Wako Pure Chemical Corporation). hPBMCs were purchased from Cellular Technology Limited and cultured in a humidified atmosphere containing 5% CO₂ at 37°C for overnight resting after thawing using a CTL-Test medium (Cellular Technology Limited). The medium was used after addition of 2 mM L-glutamine (Thermo Fisher Scientific). Meanwhile, when inducing activation, ImmunoCult-XF T Cell Expansion Medium (STEM CELL Technologies) was used and cultured in a humidified atmosphere containing 5% CO₂ at 37°C The medium was used after addition of 3 ng/mL human IL-2 (PeproTech).

### T cell activation marker measurement

Human Peripheral Blood Mononuclear Cells (hPBMCs) from healthy donors were thawed out according to the vendor's (Cellular Technology Limited) instructions, collected for resting overnight, and counted before use. Target tumor cells were plated in a 24-well flat bottom plate at a density of 8 × 10⁴ cells/well for 4 hours. hPBMCs were added in the presence and absence of target tumor cells (effector : target ratio 10:1). Next, 53L10-M13 and Tb535H were added at varying concentrations in a range of 0.001 pM to 10 nM in the presence of target tumor cells and a range of 0.01 nM to 1,000 nM in the absence of target tumor cells and incubated at 37°C for 24 or 48 hours. For staining of T cell activation markers, floating cells were collected from the plate after 24 and 48 hours, respectively. Cells collected 24 hours later were stained with the FITC anti-human CD3 antibody (Biolegend) and APC anti-human CD69 antibody (Biolegend). Cells collected 48 hours later were stained with the FITC anti-human CD3 antibody (Biolegend) and APC anti-human CD25 antibody (Biolegend). Measurement was performed using FACSLyric (BD biosciences) to measure the expression of CD3 and CD69 after 24 hours and the expression of CD3 and CD25 after 48 hours. Analysis was performed using Flow jo 10.8.1 (BD biosciences) analysis software. The percentage of CD69-positive cells in the CD3-positive cell population after 24 hours (Figure 18B) and the percentage of CD25-positive cells in the CD3-positive cell population after 48 hours (Figure 18C) were calculated. Graph creation and calculation of EC₅₀, Eₘₐₓ, and 95% confidence interval (CI) were performed using the statistical software Prism (GraphPad Software).

### Cytokine production quantification

The secretion of human TNFα, human IL-2, human IL-6, and human interferon γ in the supernatant of the co-culture of tumor cells and hPBMCs in the presence of 53L10-M13 and Tb535H was evaluated by ELISA. The culture supernatant 48 hours after the T cell activation test was centrifuged. Cytokines were quantified according to the producer's recommendations using the BD OptEIA^{™} Human TNF ELISA Set (BD bioscience) for human TNFα, BD OptEIA^{™} Human IL-2 ELISA Set (BD bioscience) for human IL-2, and the Human IL-6 ELISA Kit (R&D Systems) for human IL-6, and Human IFN-gamma ELISA Kit (R&D Systems) for human IFN-γ. Concentration values were converted to pg/mL.

### Cytotoxicity assay using flow cytometer

Human Peripheral Blood Mononuclear Cells (hPBMCs) from healthy donors were thawed out according to the vendor's (CTL) instructions, collected for resting overnight, and counted before use. Activated PBMCs were further cultured with Dynabeads Human T-activator CD3/CD28 (Thermo fisher scientific) for 3 days, collected for resting overnight without magnetic beads, and counted before use. Target tumor cells were labeled using the PKH67 Green Fluorescent Cell Linker Kit (Sigma-Aldrich) according to the producer's recommendations (25°C, 3 min for labeling reaction) and then plated in a 48-well flat bottom plate at a density of 4 × 10⁴ cells/well for 4 hours. hPBMCs were added thereto so that the effector : target ratio was 10:1 for hPBMCs without activation treatment, and the effector : target ratio was 1:1 for activated hPBMCs. Next, 53L10-M13 and Tb535H were added at varying concentrations and incubated at 37°C for 48 hours. After 48 hours, cells were collected, dead cells were stained with PI (Thermo Fisher Scientific), and the number of PKH67-positive PI-negative cells was counted using FACSLyric (BD Biosciences). Analysis was performed using Flow jo 10.8.1 (BD biosciences) analysis software for calculating Cytotoxicity (%) = (1-(Percentage of PKH67-positive PI-negative cell population when activated PBMCs and each construct were added at each concentration) / (Percentage of PKH67-positive PI-negative cell population when only activated PBMCs were added to A549)). Graph creation and calculation of EC₅₀, Eₘₐₓ, and 95% confidence interval (CI) were performed using the statistical software Prism (GraphPad Software).

### Industrial Applicability

According to the present invention, it is possible to provide a fusion protein or the like as a multispecific tribody whose anti-tumor efficacy is significantly increased as compared with the fusion protein Tb535H which is a conventional bispecific tribody. The fusion protein according to the present invention is a trispecific tribody that not only has ability to bind to 5T4 and a CD3 receptor complex, but also has ability to bind to PD-1, PD-L1 or LAG3 (that is, it also has an immune checkpoint inhibitory activity), and can exert excellent anti-tumor efficacy.

### Sequence Listing Free Text

SEQ ID NOS 1 to 95 and 106 to 109: Recombinant peptides Sequence Listing

## Claims

1. A fusion protein comprising two different first and second chains,
wherein the first chain comprises two antibody variable regions VH1 and VH2, one antibody variable region VL2, and one antibody constant region CH1 or CL,
the second chain comprises two antibody variable regions VL1 and VL3, one antibody variable region VH3, and one antibody constant region CL or CH1,
the first and second chains contain a heterodimeric interaction, and
a VH1/VL1 binding domain, a VH2/VL2 binding domain, and a VH3/VL3 binding domain, which are three combinatorial VH/VL binding domains formed within the fusion protein, have any binding ability selected from the following (provided that the three combinatorial VH/VL binding domains have different binding abilities from one another):
(i) ability to bind to 5T4;
(ii) ability to bind to a CD3 receptor complex; and
(iii) ability to bind to PD-1, PD-L1, or LAG3.

2. The fusion protein according to claim 1, which is a heterodimeric protein comprising the first chain and the second chain.

3. The fusion protein according to claim 1, wherein the two different chains comprise:
a) a first chain:
VH (5T4)-CH1-L1-VH (CD3)-L2-VL (CD3) or
VH (5T4)-CH1-L1-VL (CD3)-L2-VH (CD3) combined with a second chain:
VL (5T4)-CL-L1-VH (X)-L2-VL (X) or
VL (5T4)-CL-L1-VL (X)-L2-VH (X);
b) a first chain:
VH (5T4)-CH1-L1-VH (X)-L2-VL (X) or
VH (5T4)-CH1-L1-VL (X)-L2-VH (X) combined with a second chain:
VL (5T4)-CL-L1-VH (CD3)-L2-VL (CD3) or
VL (5T4)-CL-L1-VL (CD3)-L2-VH (CD3);
c) a first chain:
VL (5T4)-CH1-L1-VH (CD3)-L2-VL (CD3) or
VL (5T4)-CH1-L1-VL (CD3)-L2-VH (CD3) combined with a second chain:
VH (5T4)-CL-L1-VH (X)-L2-VL (X) or
VH (5T4)-CL-L1-VL (X)-L2-VH (X);
d) a first chain:
VL (5T4)-CH1-L1-VH (X)-L2-VL (X) or
VL (5T4)-CH1-L1-VL (X)-L2-VH (X) combined with a second chain:
VH (5T4)-CL-L1-VH (CD3)-L2-VL (CD3) or
VH (5T4)-CL-L1-VL (CD3)-L2-VH (CD3);
e) a first chain:
VH (CD3)-CH1-L1-VH (5T4)-L2-VL (5T4) or
VH (CD3)-CH1-L1-VL (5T4)-L2-VH (5T4) combined with a second chain:
VL (CD3)-CL-L1-VH (X)-L2-VL (X) or
VL (CD3)-CL-L1-VL (X)-L2-VH (X);
f) a first chain:
VH (CD3)-CH1-L1-VH (X)-L2-VL (X) or
VH (CD3)-CH1-L1-VL (X)-L2-VH (X) combined with a second chain:
VL (CD3)-CL-L1-VH (5T4)-L2-VL (5T4) or
VL (CD3)-CL-L1-VL (5T4)-L2-VH (5T4);
g) a first chain:
VL (CD3)-CH1-L1-VH (5T4)-L2-VL (5T4) or
VL (CD3)-CH1-L1-VL (5T4)-L2-VH (5T4) combined with a second chain:
VH (CD3)-CL-L1-VH (X)-L2-VL (X) or
VH (CD3)-CL-L1-VL (X)-L2-VH (X);
h) a first chain:
VL (CD3)-CH1-L1-VH (X)-L2-VL (X) or
VL (CD3)-CH1-L1-VL (X)-L2-VH (X) combined with a second chain:
VH (CD3)-CL-L1-VH (5T4)-L2-VL (5T4) or
VH (CD3)-CL-L1-VL (5T4)-L2-VH (5T4);
i) a first chain:
VH (X)-CH1-L1-VH (5T4)-L2-VL (5T4) or
VH (X)-CH1-L1-VL (5T4)-L2-VH (5T4) combined with a second chain:
VL (X)-CL-L1-VH (CD3)-L2-VL (CD3) or
VL (X)-CL-L1-VL (CD3)-L2-VH (CD3);
j) a first chain:
VH (X)-CH1-L1-VH (CD3)-L2-VL (CD3) or
VH (X)-CH1-L1-VL (CD3)-L2-VH (CD3) combined with a second chain:
VL (X)-CL-L1-VH (5T4)-L2-VL (5T4) or
VL (X)-CL-L1-VL (5T4)-L2-VH (5T4);
k) a first chain:
VL (X)-CH1-L1-VH (5T4)-L2-VL (5T4) or
VL (X)-CH1-L1-VL (5T4)-L2-VH (5T4) combined with a second chain:
VH (X)-CL-L1-VH (CD3)-L2-VL (CD3) or
VH (X)-CL-L1-VL (CD3)-L2-VH (CD3); or
l) a first chain:
VL (X)-CH1-L1-VH (CD3)-L2-VL (CD3) or
VL (X)-CH1-L1-VL (CD3)-L2-VH (CD3) combined with a second chain:
VH (X)-CL-L1-VH (5T4)-L2-VL (5T4) or
VH (X)-CL-L1-VL (5T4)-L2-VH (5T4)
[where in a) to l) above,
VH and VL are antibody variable regions,
VH (5T4) and VL (5T4) are antibody variable regions for 5T4,
VH (CD3) and VL (CD3) are antibody variable regions for the CD3 receptor complex,
VH (X) and VL (X) are antibody variable regions for PD-1, PD-L1, or LAG3, CH1 and CL are antibody constant regions, and
L1 and L2 are linkers].

4. The fusion protein according to claim 1, wherein amino acid sequences of complementarity determining regions (CDRs) 1, 2, and 3 in VH1, VH2, and VH3 consist respectively of:
amino acid sequences set forth in SEQ ID NOS: 3, 4, and 5;
amino acid sequences set forth in SEQ ID NOS: 9, 10, and 11;
amino acid sequences set forth in SEQ ID NOS: 24, 25, and 26 or 27;
amino acid sequences set forth in SEQ ID NOS: 37, 38, and 39;
amino acid sequences set forth in SEQ ID NOS: 47, 48 or 49, and 50; or
amino acid sequences set forth in SEQ ID NOS: 58, 59 or 49, and 60, and
wherein amino acid sequences of CDRs 1, 2, and 3 in VL1, VL2, and VL3 consist respectively of:
amino acid sequences set forth in SEQ ID NOS: 19, 20, and 21;
amino acid sequences set forth in SEQ ID NOS: 14, 15, and 16;
amino acid sequences set forth in SEQ ID NOS: 29, 30, and 31, 32, or 33;
amino acid sequences set forth in SEQ ID NOS: 41, 42, and 43;
amino acid sequences set forth in SEQ ID NOS: 52, 53, and 54; or
amino acid sequences set forth in SEQ ID NOS: 62, 63, and 64.

5. The fusion protein according to claim 1, wherein the amino acid sequences of VH1, VH2, and VH3 differ from each other, and each consist of an amino acid sequence set forth in SEQ ID NO: 88, 90, 8, 92, 23, 78, 46, 81, 57, 109, 82, 83, 84, or 36, comprise the amino acid sequence, or have at least 90% identity with the amino acid sequence, and
wherein the amino acid sequences of VL1, VL2, and VL3 differ from each other, and each consist of an amino acid sequence set forth in SEQ ID NO: 89, 91, 13, 93, 28, 79, 80, 51, 61, 85, 86, 87, or 40, comprise the amino acid sequence, or have at least 90% identity with the amino acid sequence.

6. The fusion protein according to claim 3, wherein amino acid sequences of complementarity determining regions (CDRs) 1, 2, and 3 in VH (5T4) consist respectively of amino acid sequences set forth in SEQ ID NOS: 3, 4, and 5, and
wherein amino acid sequences of CDRs 1, 2, and 3 in VL(5T4) consist respectively of:
amino acid sequences set forth in SEQ ID NOS: 19, 20, and 21.

7. The fusion protein according to claim 3, wherein amino acid sequences of complementarity determining regions (CDRs) 1, 2, and 3 in VH (CD3) consist respectively of amino acid sequences set forth in SEQ ID NOS: 3, 10, and 11, and
wherein amino acid sequences of CDRs 1, 2, and 3 in VL(CD3) consist respectively of: amino acid sequences set forth in SEQ ID NOS: 14, 15, and 16.

8. The fusion protein according to claim 3, wherein amino acid sequences of complementarity determining regions (CDRs) 1, 2, and 3 in VH (X) (where X is PD-1) consist respectively of amino acid sequences set forth in SEQ ID NOS: 24, 25, and 26 or 27, and
wherein amino acid sequences of CDRs 1, 2, and 3 in VL (X) (where X is PD-1) consist respectively of amino acid sequences set forth in SEQ ID NOS: 29, 30, and 31, 32, or 33.

9. The fusion protein according to claim 3, wherein amino acid sequences of complementarity determining regions (CDRs) 1, 2, and 3 in VH (X) (where X is PD-L1) consist respectively of amino acid sequences set forth in SEQ ID NOS: 47, 48 or 49, and 50, and
wherein amino acid sequences of CDRs 1, 2, and 3 in VL (X) (where X is PD-L1) consist respectively of amino acid sequences set forth in SEQ ID NOS: 52, 53, and 54.

10. The fusion protein according to claim 3, wherein amino acid sequences of complementarity determining regions (CDRs) 1, 2, and 3 in VH (X) (where X is PD-L1) consist respectively of amino acid sequences set forth in SEQ ID NOS: 58, 59 or 49, and 60, and
wherein amino acid sequences of CDRs 1, 2, and 3 in VL (X) (where X is PD-L1) consist respectively of amino acid sequences set forth in SEQ ID NOS: 62, 63, and 64.

11. The fusion protein according to claim 3, wherein amino acid sequences of complementarity determining regions (CDRs) 1, 2, and 3 in VH (X) (where X is LAG3) consist respectively of amino acid sequences set forth in SEQ ID NOS: 37, 38, and 39, and
wherein amino acid sequences of CDRs 1, 2, and 3 in VL (X) (where X is LAG3) consist respectively of amino acid sequences set forth in SEQ ID NOS: 41, 42, and 43.

12. The fusion protein according to claim 3, wherein the amino acid sequence of VH (5T4) consists of an amino acid sequence set forth in SEQ ID NO: 88 or 90, comprises the amino acid sequence, or has at least 90% identity with the amino acid sequence, and
wherein the amino acid sequence of VL (5T4) consists of an amino acid sequence set forth in SEQ ID NO: 89 or 91, comprises the amino acid sequence, or has at least 90% identity with the amino acid sequence.

13. The fusion protein according to claim 3, wherein the amino acid sequence of VH (CD3) consists of an amino acid sequence set forth in SEQ ID NO: 8 or 92, comprises the amino acid sequence, or has at least 90% identity with the amino acid sequence, and
wherein the amino acid sequence of VL (CD3) consists of an amino acid sequence set forth in SEQ ID NO: 13 or 93, comprises the amino acid sequence, or has at least 90% identity with the amino acid sequence.

14. The fusion protein according to claim 3, wherein the amino acid sequence of VH (X) (where X is PD-1) consists of an amino acid sequence set forth in SEQ ID NO: 23 or 78, comprises the amino acid sequence, or has at least 90% identity with the amino acid sequence, and
wherein the amino acid sequence of VL (X) (where X is PD-1) consists of an amino acid sequence set forth in SEQ ID NO: 28, 79, or 80, comprises the amino acid sequence, or has at least 90% identity with the amino acid sequence.

15. The fusion protein according to claim 3, wherein the amino acid sequence of VH (X) (where X is PD-L1) consists of an amino acid sequence set forth in SEQ ID NO: 46 or 81, comprises the amino acid sequence, or has at least 90% identity with the amino acid sequence, and
wherein the amino acid sequence of VL (X) (where X is PD-L1) consists of an amino acid sequence set forth in SEQ ID NO: 51, comprises the amino acid sequence, or has at least 90% identity with the amino acid sequence.

16. The fusion protein according to claim 3, wherein the amino acid sequence of VH (X) (where X is PD-L1) consists of an amino acid sequence set forth in SEQ ID NO: 57, 109, 82, 83, or 84, comprises the amino acid sequence, or has at least 90% identity with the amino acid sequence, and
wherein the amino acid sequence of VL (X) (where X is PD-L1) consists of an amino acid sequence set forth in SEQ ID NO: 61, 85, 86, or 87, comprises the amino acid sequence, or has at least 90% identity with the amino acid sequence.

17. The fusion protein according to claim 3, wherein the amino acid sequence of VH (X) (where X is LAG3) consists of an amino acid sequence set forth in SEQ ID NO: 36, comprises the amino acid sequence, or has at least 90% identity with the amino acid sequence, and
wherein the amino acid sequence of VL (X) (where X is LAG3) consists of an amino acid sequence set forth in SEQ ID NO: 40, comprises the amino acid sequence, or has at least 90% identity with the amino acid sequence.

18. The fusion protein according to claim 1, wherein the first chain consists of an amino acid sequence set forth in SEQ ID NO: 1, comprises the amino acid sequence, or has at least 90% identity with the amino acid sequence, and
wherein the second chain consists of an amino acid sequence set forth in SEQ ID NO: 17, 34, 44, 55, or 107 comprises the amino acid sequence, or has at least 90% identity with the amino acid sequence.

19. The fusion protein according to claim 3, wherein the first chain, VH (5T4)-CH1-L1-VH (CD3)-L2-VL (CD3), consists of an amino acid sequence set forth in SEQ ID NO: 1, comprises the amino acid sequence, or has at least 90% identity with the amino acid sequence, and
wherein the second chain, VL (5T4)CL-L1-VH (X)-L2-VL (X), consists of an amino acid sequence set forth in SEQ ID NO: 17, 34, 44, 55, or 107 comprises the amino acid sequence, or has at least 90% identity with the amino acid sequence.

20. The fusion protein according to claim 1 or 3, which is a trispecific antibody against (a) 5T4, (b) CD3 receptor complex, and (c) PD-1, PD-L1, or LAG3.

21. The fusion protein according to claim 1 or 3, which has anti-tumor activity.

22. The fusion protein according to claim 21, wherein the tumor expresses 5T4 in tumor cells or cancer cells.

23. The fusion protein according to claim 22, wherein the tumor is at least one selected from the group consisting of human mesothelioma, human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.

24. A polynucleotide encoding the fusion protein according to claim 1 or 3.

25. A vector comprising the polynucleotide according to claim 24.

26. A transformant obtained by introducing the vector according to claim 25 into a host cell.

27. A method for producing the fusion protein according to claim 1 or 3, which comprises culturing the transformant according to claim 26 and collecting the fusion protein from the obtained culture.

28. A pharmaceutical composition comprising the fusion protein according to claim 1 or 3.

29. The pharmaceutical composition according to claim 28, which is used for treating, preventing, or diagnosing a tumor.

30. The pharmaceutical composition according to claim 29, wherein the tumor expresses 5T4 in tumor cells or cancer cells.

31. The pharmaceutical composition according to claim 30, wherein the tumor is at least one selected from the group consisting of human mesothelioma, human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.

32. A method for treating, preventing, or diagnosing a tumor, which comprises administering the pharmaceutical composition according to claim 28 to a subject.

33. The method according to claim 32, wherein the tumor expresses 5T4 in tumor cells or cancer cells.

34. The method according to claim 33, wherein the tumor is at least one selected from the group consisting of human mesothelioma, human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.

35. A kit for treating, preventing, or diagnosing a tumor, which comprises the fusion protein according to claim 1 or 3.

36. The kit according to claim 35, wherein the tumor expresses 5T4 in tumor cells or cancer cells.

37. The kit according to claim 36, wherein the tumor is at least one selected from the group consisting of human mesothelioma, human lung cancer, human gastric cancer, human colorectal cancer, human esophageal cancer, human endometrial cancer, human ovarian cancer, human cervical cancer, human choriocarcinoma, human placental site trophoblastic tumor, human bladder cancer, human breast cancer, human pancreatic cancer, human prostate cancer, human kidney cancer, human head and neck cancer, and human nonseminoma germ cell tumor.
